# EUROPÄISCHE PATENTSCHRIFT

(11) **EP 0 808 838 B1**
(45) Veröffentlichungstag und Bekanntmachung des Hinweises auf die Patenterteilung: **22.10.2003**
(21) Anmeldenummer: 97107623.7
(22) Anmeldetag: 09.05.1997
(51) Int. Cl.: C07D 277/62, C07D 417/12, A61K 31/41

(54) **Schwefel enthaltende heterocyclische Bradykinin-Antagonisten, Verfahren zu ihrer Herstellung und ihre Verwendung**
Sulfur-containing heterocyclic bradykinin antagonists, processes for their production and use thereof
Antagonistes de bradykinine soufrés, procédé pour leur préparation et leur utilisation

(30) Priorität: 22.05.1996 DE 19620508
(43) Veröffentlichungstag der Anmeldung: 26.11.1997
(73) Patentinhaber: HOECHST AKTIENGESELLSCHAFT, 65929 Frankfurt am Main (DE)
(72) Erfinder: Wagner, Adalbert, Dr., 86368 Gersthofen (DE); Heitsch, Holger, Dr., 55252 Mainz-Kastel (DE); Nölken, Gerhard, Dr., 65843 Sulzbach (DE); Wirth, Klaus, Dr., 65830 Kriftel (DE); Schölkens, Bernward, Prof. Dr., 65779 Kelkheim (DE)

(56) Entgegenhaltungen:
- EP-A- 0 299 457
- EP-A- 0 596 406
- EP-A- 0 707 852
- WO-A-95/06469
- WO-A-96/12491
- WO-A-96/13485
- US-A- 4 863 923

## Beschreibung

Aus WO 96/13485, EP-A 622 361, US 5,212,182, US 5,126,165 und US 5,438,064 sind die O- und N-substituierte Chinoline und ihre Verwendung als Bradykinin-Rezeptorantagonisten bekannt. WO 95/06469 beschreibt Aminoaralkyloxybenzothiazole, die einen proliferativen Effekt zeigen und als Chemotherapeutica verwendet werden können.

Die vorliegende Erfindung betrifft schwefelenthaltende heterocyclische Verbindungen mit verbesserter Pharmakokinetik.

Die Verbindungen werden durch Formel I beschrieben 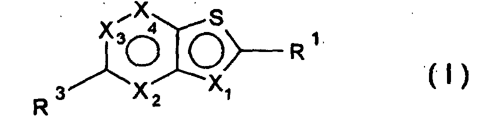 worin die Symbole folgende Bedeutungen haben:
a) einer der Reste X₁, X₂ oder X₃
   steht für C-O-R² und die jeweils anderen X₁, X₂, X₃ und X₄ sind dann gleich oder verschieden
   1. N
   2. CR¹;
b) R¹ und R³ gleich oder verschieden
   1. H
   2. Halogen
   3. (C₁-C₆)-Alkyl
   4. O-R⁶
   5. S-R⁶
   6. NHR⁶
   7. (C₆-C₁₂)-Aryl
   8. (C₆-C₁₂)-Aryl-(C₁-C₃)-Alkyl
   9. C(O)-OR⁶
   10. C(O)-H
   11. (C₂-C₅)-Alkenyl
   12. NO₂
   13. SO₃R⁷
   14. CN
   15. C(O)-NHR⁸
   wobei 3., 7., 8., 11. gegebenenfalls mit einer oder mehreren Gruppen wie C(O)-(O)ₒ-(C₁-C₅)-Alkyl, OR⁶, SR⁷, NO₂, CN, NHR⁸, Halogen substituiert sein können;
c) R² ein Rest der Formel (II) 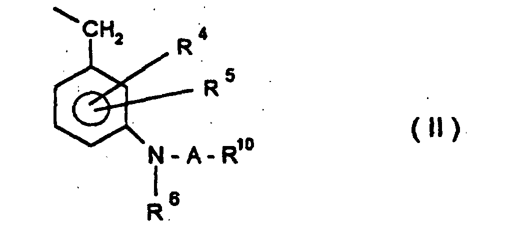 bedeutet;
d) R⁴ und R⁵ gleich oder verschieden
   1. H
   2. Halogen
   3. OR⁶
   4. SR⁶
   5. CN
   6. (C₁-C₅)-Alkyl bedeuten;
e) R⁶, R⁷ und R⁸ gleich oder verschieden
   1. H
   2. (C₁-C₅)-Alkyl
   3. (C₃-C₅)-Alkenyl
   4. (C₆-C₁₂)-Aryl-(C₁-C₃)-Alkyl;
   5. (C₃-C₁₀)-Cycloalkyl,
   6. (C₃-C₁₀)-Cycloalkyl-(C₁-C₃)-Alkyl;
   7. C(O)-(O)ₒ-(C₁-C₅)-Alkyl,
   8. C(O)-(NH)ₒ-(C₁-C₅)-Alkyl;
f) A eine Aminocarbonsäure ausgewählt aus der Gruppe Methionin, Alanin, Phenylalanin, 2-Chlorphenylalanin, 3-Chlorphenylalanin, 4-Chlorphenylalanin, 2-Fluorphenylalanin, 3-Fluorphenylalanin, 4-Fluorphenylalanin, Tyrosin, O-Methyltyrosin, β-(2-Thienyl)alanin, Glycin, Cyclohexylalanin, Leucin, Isoleucin, Valin, Norleucin, Phenylglycin, Serin, Cystein, Aminopropionsäure oder Aminobuttersäure;
g) R⁹
   1. H
   2. C(O)-(O)ₒ-(C₁-C₅)-Alkyl
   3. C(O)-(O)ₒ-(C₁-C₃)-Alkyl-(C₆-C₁₀)Aryl;
h) R¹⁰
   1. -C(O)-D-E
   2. -C(S)-D-E
   3. -SO₂-D-E
   4. Wasserstoff;
i) D
   1. (C₂-C₅)-Alkendiyl
   2. (C₁-C₈)-Alkandiyl
   3. -(CH₂)ₙ-Yₒ-(CH₂)ₘ-
   4. (C₃-C₁₀)-Cycloalkandiyl
   5. (C₃-C₁₀)-Cycloalkyl-(C₁-C₃)-alkandiyl
   6. (C₃-C₁₀)-Cycloalkendiyl
   7. (C₃-C₁₀)-Cycloalkenyl-(C₁-C₃)-alkandiyl
   wobei 1.-7. gegebenenfalls mit einer oder mehreren Gruppen wie z.B. OR⁶, NO₂, CN, CO₂R⁷, NR⁸R⁹, SO₂R⁶, SO₂NR⁸R⁹, SO₃R⁷ oder C(O)-NR⁸R⁹ substituiert sein kann;
j) E
   1. H
   2. (C₆-C₁₀)-Aryl,
   3. (C₁-C₉)-Heteroaryl,
   wobei 2. und 3. gegebenenfalls mit einer oder mehreren Gruppen substituiert sein können wie z.B. NR⁸R⁹, CN, CO₂R⁶, SO₃R⁷, NO₂, SO₂NR⁸R⁹, SO₂R⁶, O-(C₁-C₅)-Alkyl, S-(C₁-C₅)-Alkyl, (C₁-C₅)-Alkyl, (C₂-C₅)-Alkenyl, wobei O-(C₁-C₅)-Alkyl und (C₁-C₅)-Alkyl gegebenenfalls teilweise oder vollständig mit Halogen substituiert sein können;
k) Y
   1. O
   2. S
   3. NR⁸;
l) n und m gleich oder verschieden eine Zahl 0-6;
m) o 0, 1; sowie deren physiologisch verträglichen Salze.

Alkyl und Alkenyl können geradkettig oder verzweigt sein. Entsprechendes gilt für davon abgeleitete Reste wie z.B. Alkoxy.

Alkenyl steht für einfach oder mehrfach ungesättigte Verbindungen wie z.B. 1,4-Butadienyl, 8,11-Heptadienyl, 8,11,14-Heptatrienyl, Butenyl. Entsprechendes gilt für Cycloalkenyl.
Cycloalkyl steht für mono- oder bicyclische Verbindungen wie z.B. Cyclopropyl, Cyclopentyl, Cyclohexyl, Bicyclononyl. Entsprechendes gilt für Cycloalkenyl.

(C₆-C₁₂)-Aryl ist beispielsweise Phenyl, Naphthyl oder Biphenylyl, vorzugsweise Phenyl. Entsprechendes gilt auch für davon abgeleitete Reste,wie z.B. Aralkyl.

Halogen (Hal) steht für Fluor, Chlor, Brom oder Jod, vorzugsweise für Chlor oder Fluor.

Unter (C₁-C₉)-Heteroaryl werden Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Desweiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl, Benzopyranonyl, Coumarinyl, Pyranonyl, Furandionyl.

Unter physiologisch verträglichen Salzen von Verbindungen der Formel (I) versteht man sowohl deren organische als auch anorganische Salze, wie sie in Remington's Pharmaceutical Sciences (A.R. Gennard Editior, Mack Publishing Co., Easton PA, 17. Auflage, Seite 1418 (1985)) beschrieben sind. Aufgrund der physikalischen und chemischen Stabilität und der Löslichkeit sind saure Gruppen unter anderen Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Bevorzugt sind Verbindungen der Formel (I), worin
a) R¹ und R³ gleich oder verschieden
   1. H
   2. (C₁-C₆)-Alkyl
   3. O-R⁶
   4. S-R⁶
   5. NHR⁶
   6. (C₂-C₅)-Alkenyl
   7. C(O)-OR⁶
   8. C(O)-H
   9. NO₂
   10. CN
   11. C(O)-NHR⁸
   wobei 2. und 6. gegebenenfalls mit einem oder mehreren Gruppen wie z.B. Halogen, CO₂R⁶, NHR⁸ substituiert sein können;
b) R⁶, R⁷ und R⁸ gleich oder verschieden
   1. H
   2. (C₁-C₅)-Alkyl
   3. (C₆-C₁₀)-Aryl-(C₁-C₃)-Alkyl bedeuten;
   und die übrigen Reste und Variablen wie oben definiert sind.

Besonders bevorzugt sind Verbindungen der Formel (I), worin
a) R¹ und R³ gleich oder verschieden
   1. H
   2. (C₁-C₄)-Alkyl
   3. NH-(C₁-C₅)Alkyl
   4. O-(C₁-C₅)Alkyl
   5. S-(C₁-C₅)-Alkyl
   6. C(O)-H
   7. CO₂R⁶
   8. (C₂-C₃)-Alkenyl
   wobei 2.-5. und 8. mit einem oder mehreren Resten wie z.B. Halogen, CO₂R⁶, NHR⁸ substituiert sein können, bedeuten;
b) A Leucin, Isoleucin, Valin, Alanin, Methionin, Glycin, Serin, Aminopropionsäure, Aminobuttersäure bedeutet;
und die übrigen Reste und Variablen wie oben definiert sind.

Die Erfindung betrifft weiterhin ein Verfahren zur Herstellung von Verbindungen der Formel (I), das dadurch gekennzeichnet ist, daß man
a₁)
   1. eine Verbindung der Formel (III) 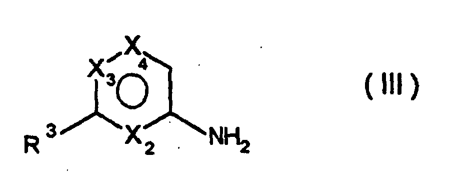 worin R³, X₂, X₃ und X₄ wie oben in Formel I definiert sind, zuerst mit aktivierten Carbonsäurederivaten, bevorzugt deren Säurechloriden unter Verwendung einer Hilfsbase, bevorzugt Triethylamin oder Diisopropylethylamin, bei Temperaturen zwischen 0-20°C acyliert,
   2. die so erhaltene Verbindung der Formel (IV) 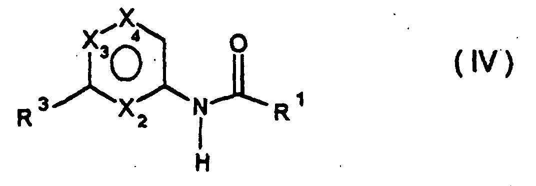 mit Laweson's Reagenz oder bevorzugt P₂S₁₀ in Butylacetat oder anderen inerten hochsiedenden Lösungsmitteln zum Sieden erhitzt und so eine Verbindung der Formel (V) erhält, 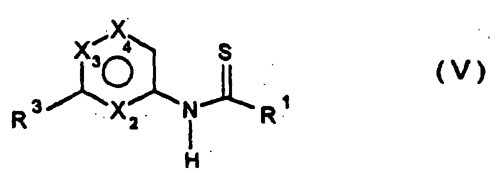 worin R¹, R³, X₂, X₃ und X₄ in Formeln IV und V wie oben in Formel I definiert sind, für X₂ oder X₃ = C-O-R² steht R² für R²' = H oder (C₁-C₅)-Alkyl, bevorzugt für Methyl oder Ethyl,
   3. die so erhaltene Verbindung (V) durch radikalische Cyclisierung mit radikalerzeugenden Reagenzien, bevorzugt K₃Fe(CN)₆ oder Br₂ in inerten Lösungsmitteln, bevorzugt H₂O, bei Temperaturen zwischen 80-110°C umsetzt und dabei eine Verbindung der Formel (VI) erhält 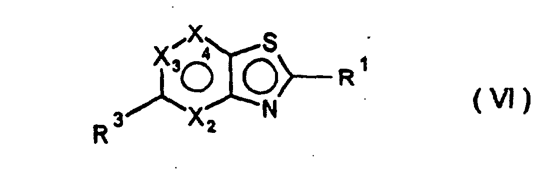 worin die Reste R¹, R³, X₂, X₃ und X₄ wie oben in Formel I definiert sind, für X₂ oder X₃ = C-O-R² steht R² für R²' = H oder (C₁-C₅)-Alkyl, bevorzugt für Methyl oder Ethyl,
   4. eine Verbindung der Formel (VI), worin X₂ oder X₃ C-O-R² bedeutet und wie unter 3. definiert ist, durch etherspaltende Reagenzien, bevorzugt BBr₃, HJ/roter Phosphor, HBr, HBr/CH₃CO₂H in inerten Lösungsmitteln oder ohne Lösungsmittel bei Temperaturen zwischen 0°C und dem Siedepunkt, zu Verbindungen der Formel (VI) umsetzt, worin X² oder X³ COR² bedeutet und R² = R²' = Wasserstoff ist;
   oder
a₂)
   1. eine Verbindung der Formel (VII) 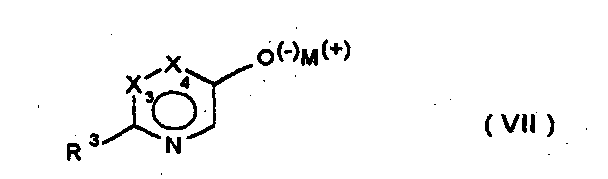 worin R³, X₃, X₄ wie oben in Formel I definiert sind und M für Kalium, Natrium oder Cäsium steht,
      durch sukzessive Behandlung mit CO₂ und anschließend NH₃ unter erhöhtem Druck und Temperaturen, bevorzugt 100 atm und 200°C, in eine Verbindung der Formel (VIII) umwandelt, 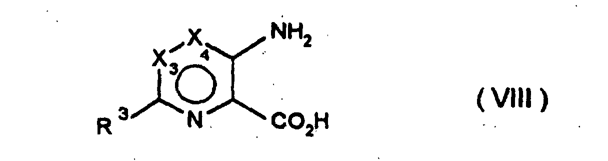 worin R³, X₃, X₄ wie oben in Formel I definiert sind,
   2. eine Verbindung der Formel (VIII) durch Diazotierung und anschließender Behandlung mit HS-CHR¹-CO₂H in eine Verbindung der Formel (IX) umwandelt, 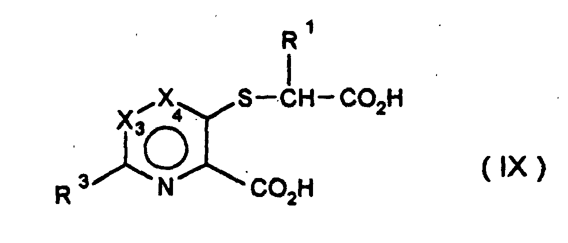 worin R¹, R³, X₃, X₄ wie oben in Formel I definiert sind,
   3. eine Verbindung der Formel (IX) durch Cyclisierung unter gleichzeitiger Decarboxylierung und Wasserabspaltung in inerten Lösungsmitteln, bevorzugt H₂O, oder ohne Lösungsmittel, bevorzugt bei Temperaturen ~ 100°C in eine Verbindung der Formel (X) überführt, 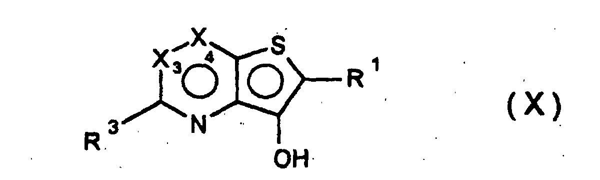
   worin R¹, R³, X₃, X₄ wie oben in Formel (I) definiert sind;
b) eine Verbindung der Formeln (VI) oder (X) 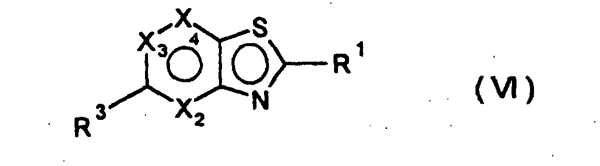 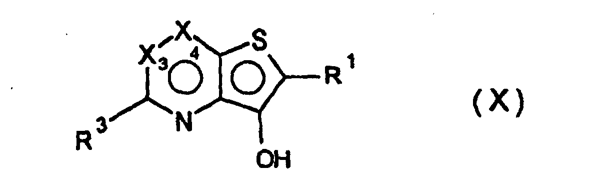 worin X₂, X₃, X₄, R¹ und R³ wie oben in Formel (I) definiert sind, im Falle von Verbindungen der Formel (VI) X₂ oder X₃ = C-O-H bedeutet, mit Cs₂CO₃ oder K₂CO₃ in einem inerten Lösungsmittel, bevorzugt DMF oder N-Methylpyrrolidin, deprotoniert und bei Raumtemperatur mit einer Verbindung der Formel (XI) 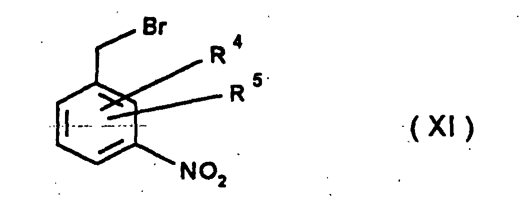 worin R⁴ und R⁵ wie oben in Formel (II) definiert sind, umsetzt;
c) die so erhaltenen Verbindungen der Formeln (XII) oder (XII') 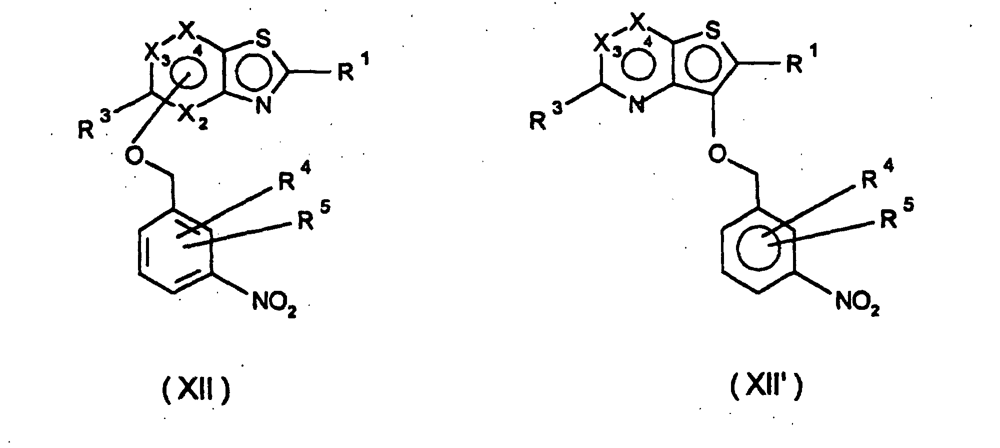 worin R¹, R³, R⁴, R⁵, X₂, X₃, X₄ wie oben in Formel (I) und (II) definiert sind, mit Hilfe von Übergangsmetallhalogeniden, bevorzugt SnCl₂, FeCl₃ zu einer Verbindung der Formeln (XIII) oder (XIII') reduziert, 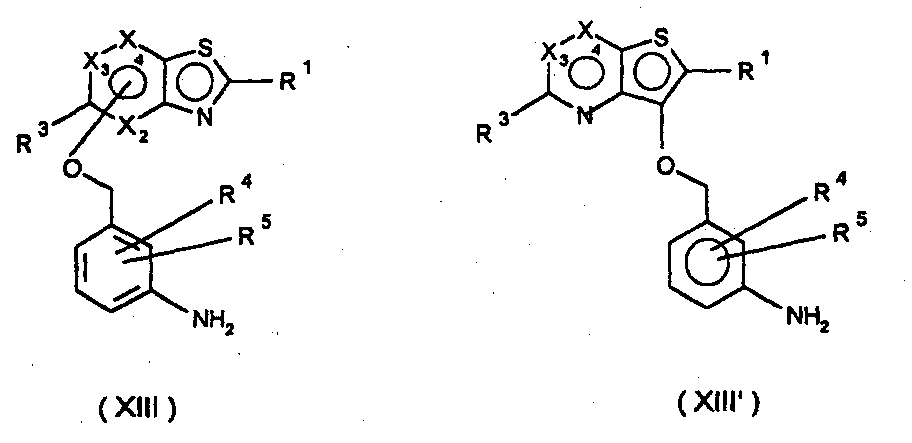 worin R¹, R³, R⁴, R⁵, X₂, X₃ und X₄ wie oben in Formeln (I) und (II) definiert sind;
d) eine Verbindung der Formeln (XIII) oder (XIII') mit aktivierten, geeignet geschützten Aminocarbonsäurederivaten von A (A-Prot), bevorzugt den Säurechloriden der Phthaloyl-geschützten Aminocarbonsäurederivate von A, in inerten Lösungsmitteln wie z.B. NMP, gegebenenfalls durch Zugabe von DMAP, umsetzt und so eine Verbindung der Formeln (XIV) oder (XIV') erhält, 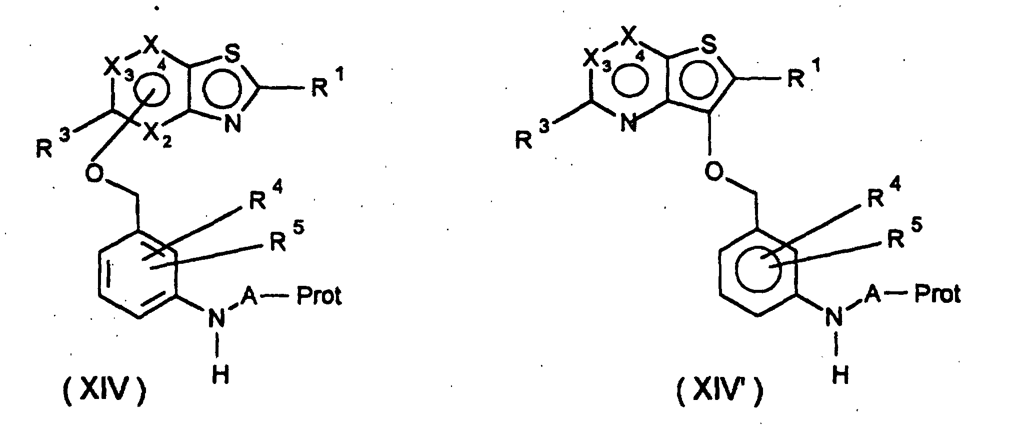 worin A, R¹, R³, R⁴, R⁵, X₂, X₃ und X₄ wie oben in Formeln (I) und (II) definiert sind, und
   Prot für eine Aminoschutzgruppe steht, wie in T.W. Greene "Protective Groups in Organic Synthesis", Verlag John Wiley, 2. Auflage 1991 beschrieben, wobei beide Protonen der Aminoschutzgruppe geschützt sind, z.B. Benzyl, Paramethoxybenzyl oder Phthaloyl;
e) eine Verbindung der Formeln (XIV) oder (XIV') nach erfolgter Einwirkung von Alkalihydriden, Alkalicarbonaten oder Alkoholaten in inerten Lösungsmitteln, bevorzugt DMF oder NMP, mit R⁶X, wobei R⁶ wie oben in Formel (I), ausgenommen R⁶=H, definiert ist und X eine Abgangsgruppe, z. B. Halogen, Mesylat oder Tosylat, bedeutet, umsetzt, wobei man eine Verbindung der Formeln (XV) oder (XV') erhält. 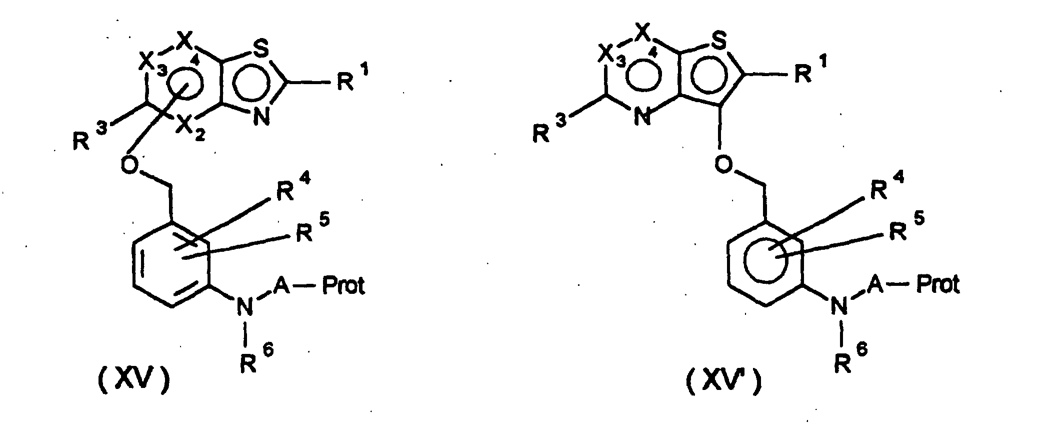 worin A, R¹, R³, R⁴, R⁵, R⁶, X₂, X₃ und X₄ wie oben in Formel (I) und (II) und Prot wie oben in Formel (XIV) definiert sind;
f) zum Entfernen der Schutzgruppe (Prot.) von der Verbindung der Formeln (XV) oder (XV'), im Falle der Phthaloylgruppe bevorzugt mit Hydrazin in Alkoholen als Lösungsmittel, bei Temperaturen zwischen Raumtemperatur und dem Siedepunkt, bevorzugt bei Raumtemperatur, umsetzt, wobei man eine Verbindung der Formeln (XVI) oder (XVI') erhält, 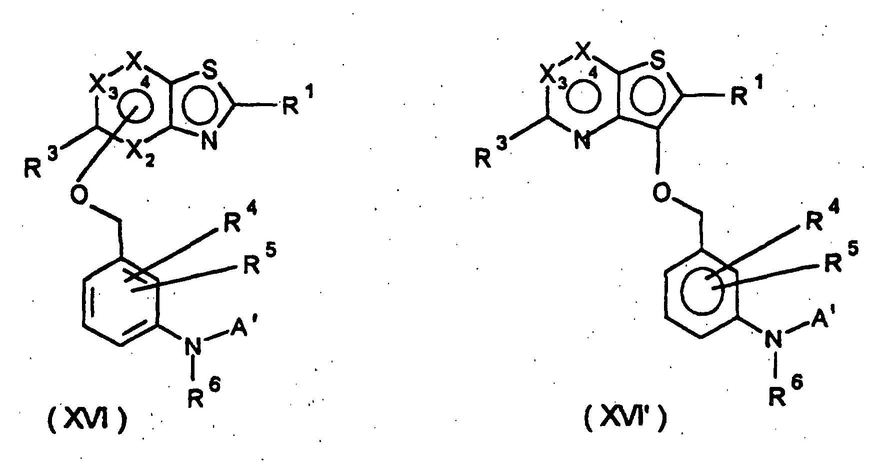 worin A, R¹, R³, R⁴, R⁵, R⁶, X₂, X₃ und X₄ wie oben in Formeln (I) und (II) und Prot wie oben in Formel (XIV) definiert sind und A' ein Rest der Aminosäure A ist.
   g₁) eine Verbindung der Formeln (XVI) oder (XVI') mit aktivierten Säurederivaten der Formeln (XVII), (XVIII) oder (XIX)

      E - D - C(O) - OH (XVII)

      E - D - C(S) - OH (XVIII)

      E - D - SO₂ - OH (XIX)
   worin D und E wie oben in Formel (II) definiert sind,
   bevorzugt deren Säurechloriden, Anhydriden oder Säuren der Formeln (XVII), (XVIII) oder (XIX), aktiviert durch Reagenzien, wie sie in der Peptidsynthese verwendet werden, umsetzt, oder
g₂) eine Verbindung der Formeln (XVI) oder (XVI') mit einem Amin oder einem Alkohol der Formel (XX)

   E - D - Z (XX)

   worin E und D wie oben definiert sind und Z OH oder NH₂ bedeuten, wobei man zunächst jedoch die Verbindungen der Formeln (XVI), (XVI') oder (XX) mit einer doppelt aktivierten Carbonylverbindung zur Bildung der Harnstoff- bzw. Urethangruppe, z.B. mit Carbodiimiden, Phosgen oder Chlorkohlensäureestern, bevorzugt Phosgen und Carbonyldiimidazol, reagieren läßt, umsetzt, bevorzugt bei Temperaturen zwischen 0°C und Raumtemperatur in inerten Lösungsmitteln, bevorzugt Dichlormethan oder Dimethoxyethan, oder
g₃) eine Verbindung der Formeln (XVI) oder (XVI') mit einem entsprechenden Isocyanat oder Isothiocyanat, bevorzugt bei Temperaturen zwischen 0°C und Raumtemperatur in inerten Lösungsmitteln, bevorzugt Dichlormethan oder Dimethoxyethan, umsetzt, und
h) die erhaltene Verbindung der Formel (I) gegebenenfalls nach bekannten Methoden in ihre physiologisch verträglichen Salze überführt.

Der Austausch von Chlor gegen Alkoxy oder den entsprechenden S-Alkylen erfolgt durch Reaktion mit den entsprechenden Alkoholaten oder Thiolaten, bevorzugt deren Alkali- oder Erdalkalisalzen in inerten Lösungsmitteln bevorzugt DMF, NMP oder dem entsprechenden Alkohol bei Temperaturen zwischen 0°C und 60°C, bevorzugt zwischen 0°C und Raumtemperatur.

Der Ersatz von Chlor gegen Cyano erfolgt durch Einwirken von Cyaniden, bevorzugt den Kupfercyaniden, in inerten hochsiedenden Lösungsmitteln, wie z.B. DMF oder NMP bei deren Siedepunkten.

Die Konversion zur Bromethylverbindung erfolgt durch Umsetzung des entsprechenden Methyl-Derivates mit N-Bromsuccinimid, Dibromhydantoin oder Brom in inerten Lösungsmitteln, bevorzugt Brombenzol oder Cyclohexan bei Temperaturen von 60 °C bis zum Siedepunkt.

Als Kupplungsreagenz können alle möglichen in der Peptidsynthese verwendeten Aktivierungsreagenzien, siehe z. B. Houben-Weyl, Methoden der organischen Chemie, Band 15/2, Georg Thieme Verlag, Stuttgart 1974, verwendet werden, insbesondere aber Carbodiimide wie z. B. N,N'-Dicyclohexylcarbodiimid, N,N'-Diisopropyl-carbodiimid oder N-Ethyl-N'-(3-dimethylaminopropyl)carbodiimid. Die Kupplung kann dabei direkt durch Addition von Carbonsäurederivat mit dem Aktivierungsreagenz und gegebenenfalls einem Zusatz wie z.B. 1-Hydroxybenzotriazol (HOBt) (W. König, R. Geiger, Chem. Ber. 103, 708 (1970)) oder 3-Hydroxy-4-oxo-3,4-dihydrobenzotriazin (HOObt) (W. König, R.Geiger, Chem. Ber. 103, 2054 (1970)) durchgeführt werden oder aber die Voraktivierung des Carbonsäurederivats als symmetrisches Anhydrid oder HOBt- bzw. HOObt-Ester kann separat erfolgen und die Lösung der aktivierten Spezies in einem geeigneten Lösungsmittel zum Amin gegeben werden.

Die Kupplung bzw. Aktivierung der Aminosäurederivate mit einem der oben genannten Aktivierungsreagenzien kann in Dimethylformamid, N-Methylpyrrolidon oder Methylenchlorid oder einer Mischung aus den genannten Lösungsmitteln durchgeführt werden.

Anstelle der Phthaloylgruppe können auch Schutzgruppen verwendet werden, die beide Protonen der Aminogruppe schützen, z.B. zwei Benzylgruppen.

Die erfindungsgemäßen Verbindungen haben einzeln oder in Kombination eine bradykinin-antagonistische Wirkung, die in verschiedenen Modellen getestet werden kann (s. Handbook of Exp. Pharmacol. Vol. 25, Springer Verlag, 1970, S. 53 - 55), so z. B. am isolierten Rattenuterus, am Meerschweinchenileum, der Jugularvene des Kaninchens oder an der isolierten Pulmonalarterie des Meerschweinchens. Die Effekte der Verbindungen der Formel (I) auf bradykinininduzierte Bronchokonstriktion und Carregeenin induziertes Pfotenödem können analog Br. J. Pharmacol. 102, 774 - 777 (1991) bestimmt werden.

Die Messung der Bindung an den Bradykinin B₂ Rezeptor vom Meerschweinchenileum ist im Folgenden beschrieben (R. B. Innis et al., Proc. Natl. Acad. Sci. USA; 17 (1981) 2630):
1. Ligand: ³H-BRADYKININ (von NEN Du Pont)
2. Pufferansätze:
   a) TES-Puffer:
      25 mM TES (SIGMA, Best.Nr.: T-4152)
      1 mM 1,10-Phenanthrolin (SIGMA; Best.Nr.: P-9375)
   b) Inkubations-Puffer:
      25 mM TES (SIGMA; Best.Nr.: T-4152)
      1 mM1,10-Phenanthrolin (SIGMA; Best.Nr.: P-9375)
      0,1% Albumin, Bovine (SIGMA; Best.Nr.: A-7906)
      140 µg/ml Bacitracin (SIGMA; Best.Nr.: B-0125)
      1 mM Dithiothreitol (SIGMA; Best.Nr.: D-0632)
      1 µM Captopril → 1-[(2S)-3-Mercapto-2-methylpropionyl]-L-prolin
   Beide Puffer werden mit 5 molarer NaOH auf pH 6,8 eingestellt.
3. Membranpräparation:
   Meerschweinchen-Ilea werden durch vorsichtiges Ausstreichen grob vom Darm-Inhalt befreit und in 0,9%iger NaCl-Lösung gesäubert.
   Die ca. 2 cm langen Ilea-Stücke werden in eiskalten TES-Puffer überführt (ca. 1g /10 ml) und mit dem Ultraturrax ca. 30 sec. lang im Eisbad homogenisiert.
   Das Homogenat wird anschließend durch 3 Lagen Gaze gefiltert und das Filtrat bei 50.000 g / 10 Minuten zentrifugiert.
   Der Überstand wird verworfen, das Pellet in dem gleichem Volumen TES-Puffer rehomogenisiert und erneut bei 50.000 g / 10 Minuten zentrifugiert.
   Das Pellet wird in Inkubations-Puffer rehomogenisiert (ca. 1 g / 5 ml) und in Kryoröhrchen, 2 ml portioniert, bei -70°C eingefroren.

   Die Proteinkonzentration der fertigen Membransuspension wird nach LOWRY bestimmt und sollte ca. 15 µg / 100 µl betragen.
4. Bindungstest:
   Alle Inkubationen werden bei Raumtemperatur für 60 Minuten auf
   Mikrotiterplatten (96 x 300 µl) in 200 µl Volumen durchgeführt. Alle Ansätze in
   Inkubations-Puffer. Dazu werden 50 µl des Radioliganden, 50 µl des zu prüfenden Präparats und 100 µl der Membransuspension nacheinander in die Vertiefungen der Mikrotiterplatte pipettiert.

   a) Sättigungsexperimente (Heiße Sättigung):
      Herstellung der ³H-Bradykinin-Lösung: Für die Sättigungsexperimente werden die Konzentrationen 0.05, 0.1, 0.2, 0.4, 0.6, 0.8, 1.0, 1.5, 2.0, 2.5 und 3.0 nMol/l, das entspricht 0.05 bis 3.0 pMol/ml, eingesetzt. Nach der Herstellung der entsprechenden Verdünnungen werden je 50 µl pro Probe vorgelegt.
      Unspezifische Bindung: Für jede Konzentration des radioaktiven Liganden muß die unspezifische Bindung bestimmt werden. Dies kann durch Zugabe einer hohen Konzentration (1-100 µMol) des nichtmarkierten Liganden, anderer Antagonisten oder Agonisten des Bradykinin-Rezeptors erreicht werden. In diesem Test wird HOE 140 (10 µMol/l) verwendet. Dazu werden 1,862 mg in 1 ml Dimethylsulfoxid (DMSO) gelöst, 1:25 mit Inkubationspuffer verdünnt und von dieser Lösung 50 µl zu den Proben in die Mikrotiterplatte gegeben. Die Reaktion wird durch die Zugabe von 100 µl der Membransuspension gestartet.
   b) Kompetitionsexperimente (IC₅₀):
      Hier werden eine feste Größe des radioaktiven Liganden (0,25 bis 0,3 nMol/l ³H-Bradykinin) und verschiedene Konzentrationen der nichtmarkierten Agonisten oder Antagonisten eingesetzt.
      Zu jeweils 50 µl der ³H-Bradykinin-Lösung werden 50 µl der zu prüfenden Präparate bzw. Standards in den Konzentrationen 10⁻⁵ bis 10⁻¹⁰ Mol/l zugegeben und die Reaktion durch Zugabe von 100 µl Membransuspension gestartet. Auch in diesem Test werden 3fach-Bestimmungen durchgeführt und drei Proben zur Bestimmung der unspezifischen Bindung mit 10 µMol/l HOE 140 inkubiert.
      Die auf Kompetition zu prüfenden Präparate werden grundsätzlich in einer Konzentration von 1 mMol/l in Dimethylsulfoxid (DMSO) gelöst, und anschließend mit DMSO weiterverdünnt. Diese Lösung wird dann 1:25 mit Inkubationspuffer verdünnt.
      Nach der Inkubation werden die Proben im Skatron Zellharvester über einen vorher mit 0,1% PEI (Polyethylenimin) angefeuchteten Whatmann GF/B Filterpapierstreifen abfiltriert und mit, je Probe, 10 ml eiskaltem TES-Puffer nachgewaschen. Die noch feuchten Filter werden in Mini-Scintillationsröhrchen ausgestanzt und mit 3 ml Scintillator aufgefüllt.
      Nach ca. 12 Stunden Einweichzeit werden die Proben kurz aufgeschüttelt und im Beta-Counter gemessen.
   c) Screening:
      Im Primär-Screening werden im allgemeinen nur 1-2 Konzentrationen des Prüfpräparats (10⁻⁵ und 10⁻⁶ Mol/l) eingesetzt. Ist bei der höchsten Konzentration eine Verdrängung des Radioliganden von 50% oder mehr nachweisbar, wird eine vollständige Analyse (Kompetitions-Experiment) mit mind. 8 Konzentrationen vorgenommen.
   4. Auswertung:
      Die Auswertung erfolgt mittels des LIGAND-Programmpakets (Mc Pherrson, Minson & Rodbard, Vertrieb: Elsevier-BIOSOFT), das die notwendigen Berechnungen zur Bestimmung von IC₅₀ und Kᵢ-Werten vornimmt. Dieses Programm führt außerdem grafische Darstellungen der Sättigungs- bzw. Verdrängungs-Kurven sowie den SCATCHARD-Plot, HILL-Plot oder HOFSTEE-Plot durch.

Die Bestimmungen der antagonistischen Wirkung auf die bradykinininduzierte Kontraktion des Meerschweinchen-Ileums erfolgt nach folgendem Protokoll:

Meerschweinchen im Gewicht von ca. 300 g (Morioth strain, ♂♀) werden durch Nackenschlag getötet und entblutet. Das Ileum wird in einer Länge von ca. 20 cm herauspräpariert, mit Tyrode-Lösung durchspült (Recordspritze) und so vom Darminhalt befreit. Nun wird es in 1,5 cm lange Abschnitte geteilt. Diese werden in 10 ml fassenden Organbädern, die mit Tyrode-Lösung gefüllt sind, fixiert und mit Dehnungsmeßstreifen (isometrische Kontraktionsmessung) verbunden. Die Vorlast beträgt 1g. Die Tyrode-Lösung wird in einem Wasserbad auf 37°C erwärmt und mit Druckluft durchperlt.
Nach einem Intervall von 30 min. wird mit dem Versuch begonnen.
Nach Aufzeichnung der biologischen Null-Linie wird pro Organbad Bradykinin in einer Endkonzentration von 4 x 10⁻⁸ mol/l zugesetzt und die Konzentration aufgezeichnet. Danach wird 3 min. mit Tyrode-Lösung gespült und nach einer Ruhepause von 20 min. wieder Bradykinin hinzugefügt. Das Maximum der Kontraktion ist erreicht (Kontrolle). Wieder spülen, Ruhepause. Nun wird der Bradykinin-Antagonist zugefügt (Einwirkungszeit 10 min.). Danach wird wieder Bradykinin zugesetzt und die nun erfolgende Kontraktion mit der Kontrolle verglichen. Die Aufzeichnung des Versuchs erfolgt auf einem Tintenschreiber.

| | | |
|---|---|---|
| Tyrode-Lösung (mM): | NaCl | 137 |
| | Glucose | 5,05 |
| | KCl | 2,68 |
| | NaHCO₃ | 11,9 |
| | NaH₂PO₄ | 0,47 |
| | MgCl₂ x 2H₂O | 0,49 |
| | CaCl₂ x 2H₂O | 0,68 |

Verstärker: TF6 V3 Fa. Fleck, Mainz
Tintenschreiber: Goerz Metrawatt SE 460, BBC
Bradykinin: Fa. Bachem

Für die Testung der erfindungsgemäßen Verbindungen an der isolierten Arteria pulmonalis werden Meerschweinchen (Dunkin Hartley) mit einem Gewicht von 400-450 g durch Genickschlag getötet.

Der Brustkorb wird geöffnet und die Arteria pulmonalis vorsichtig herauspräpariert. Das umliegende Gewebe wird sorgfältig entfernt und die Arteria pulmonalis in einem Winkel von 45° spiralig aufgeschnitten.

Der Gefäßstreifen von 2,5 cm Länge und 3-4 mm Breite wird in einem 10 ml fassenden Organbad, das mit Ringerlösung gefüllt ist, fixiert.

### Zusammensetzung der Lösung in mmol/l

| | |
|---|---|
| NaCl | 154 |
| KCl | 5,6 |
| CaCl₂ | 1,9 |
| NaHCO₃ | 2,4 |
| Glukose | 5,4 |

Die Lösung wird mit 95 % O₂ und 5 % CO₂ durchperlt und auf 37°C erwärmt. Der pH beträgt 7,4, die Vorlast am Gefäßstreifen beträgt 1,0 g.

Die isotonische Kontraktionsänderungen werden mit einem Hebelvorsatz und einem HF-Modem (Wegmesser) von Hugo Sachs erfaßt und auf einen Kompensationsschreiber (BEC, Goerz Metrawatt SE 460) registriert.

Nach 1 Stunde Äquilibrierung wird mit dem Versuch begonnen. Nachdem die Gefäßstreifen ihre maximale Empfindlichkeit gegenüber 2x10⁻⁷ mol/l Bradykinin erreicht haben - Bradykinin führt zu einer Kontraktion der Gefäßstreifen - werden die Testverbindungen in den Dosen 5x10⁻⁸ - 1x10⁻⁵ mol/l jeweils 10 Minuten einwirken gelassen und nach erneuter Gabe von Bradykinin die Abnahme des Effekts von Bradykinin gegenüber der Kontrolle verglichen.

Methodenbeschreibung zur Bestimmung des Effekts der Verbindungen der Formel (I) an der isolierten Kaninchen-Jugularvene:

Männliche Kaninchen (weiße Neuseeländer, Züchter: Möllegaard, Dänemark, 2,5 - 3,0 kg) werden durch Injektion einer Überdosis Pentobarbital-Na (1 ml Narcoren® + 0,5 ml Heparin) getötet. Die beiden Jugularis-Venen werden freipräpariert, spiralförmig aufgeschnitten und Stücke von ca. 1,5 cm Länge in gepufferte Organbäder (Krebs-Henseleit-Puffer) bei einer Vorspannung von 0,5 g eingehängt.
Nach 30 min Ruhezeit werden Kontraktionen ausgelöst durch Zugabe von Bradykinin (10⁻⁷ M), die als Ausgangswert dienen. Prüfsubstanzen werden nun in einer Konzentration von 10⁻⁵ M zugegeben. Die gezeigten Hemmwerte sind Mittelwerte (n=6).
Die angegebenen Werte zum Zeitpunkt 15 min zeigen die Hemmung der Bradykinin-induzierten Kontraktion noch in Anwesenheit der Prüfsubstanzen in der Badflüssigkeit nach 15-minütiger Inkubation. Danach wird die Bradykinin-Kontraktion durch Spülen mit bloßer Pufferlösung beendet.
Zu jedem weiteren aufgeführten Zeitpunkt wurde erneut mit Bradykinin stimuliert (in Abwesenheit der Prüfsubstanz in der Badflüssigkeit) und zur Beendigung der Kontraktion die Badflüssigkeit durch bloße Pufferlösung ersetzt.

Nach den oben beschriebenen Methoden wurde die antagonistische Wirkung der Beispiele 3, 6, 11, 12, 23, 37 ohne auf sie zu beschränken, am B₂-Rezeptor des Meerschweinchens als IC₅₀ < 1•10⁻⁷ M und an der Arteria pulmonalis des Meerschweinchens mit IC₅₀-Werten kleiner als 1 x 10⁻⁶M bestimmt.

Für die orale Anwendungsform oder zur Applikation auf die Schleimhäute werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z. B.Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Ein Präparat für die topische Anwendung kann als wäßrige oder ölige Lösung, Lotion, Emulsion oder Gelee, Salbe oder Fettsalbe oder, falls möglich, in Sprayform vorliegen, wobei gegebenenfalls durch Zusatz eines Polymers die Haftung verbessert werden kann.

Für die intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Wäßrigen intranasalen Zubereitungen können Chelatbildner, Ethylendiamin-N,N,N',N'-tetraessigsäure, Citronensäure, Weinsäure oder deren Salze zugefügt werden. Die Applikation der Nasallösungen kann mittels Dosierzerstäuber erfolgen oder als Nasaltropfen mit viskositätserhöhendem Anteil bzw. Nasengels oder Nasencremes.

Die beschriebenen Verbindungen der Formel (I) und deren pharmakologisch geeigneten Salze sind potente Bradykinin-Antagonisten. Daher liegt ihr therapeutischer Nutzen in der Behandlung und/oder der Prevention aller pathologischer Zustände, die durch Bradykinin und Bradykinin-analoge Peptide vermittelt, ausgelöst oder unterstützt werden. Dies beinhaltet u. a. Allergien, Entzündungen, Autoimmunerkrankungen, Schock, Schmerz und, mehr speziell, Asthma, Husten, Bronchitis, Rhinitis, chronisch obstruktive Pulmonarerkrankungen, Pneumonitis, septischen Schock, endotoxischen Schock, anaphylaktischen Schock, disserminierende intravaskuläre Koagulopathie, Arthritis, Rheuma, Osteoarthritis, Lumbago, entzündungsinduzierte Knochenresorption, Konjunctivitis, Iritis, Kopfschmerz, Migräne, Zahnschmerz, Rückenschmerz, krebsbedingte Schmerzen, postoperativen Schmerz, Traumata (Wunden, Verbrennungen usw.), Ausschlag, Erytheme, Ödeme, Ekzeme, Dermatitis, Zoster, Herpes, Juckreiz, Psoriasis, Flechte, entzündliche Darmerkrankungen, Hepatitis, Pankreatitis, Gastritis, Ösophagitis, Nahrungsallergien, Geschwüre, Reizdarm, Angina, Hirnödem, niedriger Blutdruck, Thrombose, Schädel-Hirn- und Wirbelsäulen-Trauma, Frühgeburt, Artherosklerose, Aszitis bei Malignom, Tumormetastasen, Hirnödem bei Tumoren, Hitzeschädigung des Gehirns, Virus-Erkrankungen und Leberzirrhose.

Da weiterhin bekannt ist, daß Bradykinin verknüpft ist mit der Freisetzung von Mediatoren wie Prostaglandinen, Leukotrienen, Tachykininen, Histamin, Thromboxanen, besitzen die Verbindungen der Formel (I) somit auch das Potential zur Behandlung und/oder Prevention von den Krankheiten, die durch diese Mediatoren hervorgerufen werden.

Die Erfindung betrifft daher auch die Verwendung von Verbindungen der Formel (I) als Heilmittel und pharmazeutische Präparate, die diese Verbindungen enthalten.

Pharmazeutische Präparate enthalten eine wirksame Menge des Wirkstoffs der Formel (I) - einzeln oder in Kombination - zusammen mit einem anorganischen oder organischen pharmazeutisch verwendbaren Trägerstoff.

Die Anwendung kann enteral, parenteral - wie z. B. subkutan, i. m. oder i. v. -, sublingual, epikutan, nasal, rektal, intravaginal, intrabukkal oder per Inhalation erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für die inhalative Anwendung können Vernebler oder Druckgaspackungen unter Verwendung inerter Trägergase benutzt werden.

Zur intravenösen, subkutanen, epikutanen oder intradermalen Applikation werden die aktiven Verbindungen oder deren physiologisch verträgliche Salze, gewünschtenfalls mit den pharmazeutisch üblichen Hilfsstoffen, beispielsweise zur Isotonierung oder pH-Einstellung sowie Lösungsvermittler, Emulgatoren, oder anderen Hilfsstoffen, in Lösung, Suspension oder Emulsion gebracht.

Sollten die Halbwertszeiten der beschriebenen Arzneistoffe in Körperflüssigkeiten unzureichend sein, ist der Einsatz von injizierbaren Retardzubereitungen sinnvoll.
Als Arzneiformen können z. B. ölige Kristallsuspensionen, Mikrokapseln, Rods oder Implantate verwendet werden, wobei die letzteren aus gewebsverträglichen Polymeren, insbesondere bioabbaubaren Polymeren, wie z. B. auf der Basis von Polymilchsäure-Polyglykolsäure-Copolymeren oder Humanalbumin aufgebaut sein können.

Ein geeigneter Dosisbereich für topische und inhalative Anwendungsformen sind Lösungen mit 0,01 - 5 mg/l, bei systemischen Applikationsformen sind 0,01 - 10 mg/kg geeignet.
Allgemein können täglich Mengen von 0,1 mg bis zu 1000 mg, bezogen auf einen Erwachsenen von 75 kg Körpergewicht, appliziert werden.

| Liste der Abkürzungen: | |
|---|---|
| AIBN | α, α'-Azobisisobutyronitril |
| DEI | Desorption Electron Impact |
| DCI | Desorption-Chemical Ionisation |
| E | Ethylacetat |
| FAB | Fast Atom Bombardment |
| DME | Dimethoxyethan |
| DMF | Dimethylformamid |
| DMAP | Dimethylaminopyridin |
| NMP | N-Methylpyrrolidon |
| H | n-Heptan |
| RT | Raumtemperatur |
| CH₂Cl₂ | Dichlormethan |
| h | Stunden |
| ESI | electron spray ionisation |
| T | Temperatur |

Die Erfindung wird durch die nachstehenden Beispiele erläutert.

### Beispiel 1

### 4-[3-(N-(3-Methoxycinnamoylglycyl)-N-methylamino)-2,6-dichlorobenzyloxy]-benzthiazol

a) 2,6-Dichloro-3-nitrobenzylbromid
   Zu 2,6-Dichloro-3-nitrotoluol (100 g, 0,48 mol) in Chlorbenzol (400 ml) wurden bei 150°C ein Gemisch aus Dibromhydantoin (70 g, 0,24 mol) und AIBN (5 g) portionsweise zugegeben. Nach 1 h wurde nochmals ein Gemisch aus Dibromhydantoin (35 g, 0,12 mol) und AIBN (2,5 g) zugegeben. Nach weiteren 1,5 h ließ man abkühlen und es wurde Ethylacetat (500 ml) zugegeben. Dieses Gemisch wurde je 1 mal mit gesättigter Na₂SO₃, Na₂CO₃ und NaCl-Lösung gewaschen, getrocknet (MgSO₄) und eingeengt, wobei die Titelverbindung als amorphes Pulver anfiel.
   R_{f} (E/H 1/1) = 0,7 MS (DEI) = 283 (M⁺)
b) 4-(2,6-Dichloro-3-nitro-benzyloxy)-benzthiazol
   Zu 4-Hydroxybenzthiazol (Helv. Chim. Acta 25 (1942) 515) (3,3 g, 21,8 mmol) und K₂CO₃ (3 g, 21,7 mmol) in DMF (50 ml) wurde die Titelverbindung gemäß Beispiel 1a) (6 g, 21,1 mmol) bei RT zugegeben. Nach 90 min wurde mit Ethylacetat (500 ml) verdünnt, 5 mal mit je 100 ml H₂O gewaschen, die organische Phase getrocknet (MgSO₄) und eingeengt. Kristallisation aus Ethylacetat liefert die Titelverbindung als amorphes Pulver.
   R_{f} (E/H 1/1) = 0,5 MS (ESI) = 355 (M+1)
c) 4-(3-Amino-2,6-dichlorobenzyloxy)-benzthiazol
   Zur Titelverbindung gemäß Beispiel 1b) (3,8 g, 10,7 mmol) in Ethylacetat (50 ml) wurde SnCl₂ x H₂O (12 g, 53 mmol) zugegeben und die Mischung auf 70°C erwärmt. Nach 1 h ließ man auf RT abkühlen und schüttete das Gemisch auf Eis (ca. 300 g). Nun wurde durch Zugabe von 2n NaOH Lösung ein pH von 13 eingestellt und in der Kälte 3 mal mit je 200 ml Ethylacetat extrahiert. Die vereinten organischen Extrakte wurden 1 mal mit gesättigter NaHCO₃-Lösung (200 ml) gewaschen, getrocknet (MgSO₄) und eingeengt und roh für den nächsten Reaktionsschritt verwendet.
   R_{f} (E/H 1/1) = 0,4 MS (ESI) = 325 (M+1)
d) 4-(2,6-Dichloro-3-phthaloylglycylamino-benzyloxy)-benzthiazol
   Ein Gemisch aus der Titelverbindung gemäß Beispiel 1c) (1,2 g, 3,7 mmol) Phthaloylglycylchlorid (1,3 g, 7 mmol), DMAP (160 mg, 1,3 mmol) und Pyridin (2 ml) in NMP (10 ml) wurde auf 50°C erwärmt. Nach 2h ließ man auf RT abkühlen und schüttete das Gemisch auf H₂O (ca. 200 ml). Der entstandene Niederschlag wurde filtriert, mit H₂O gewaschen und an der Luft getrocknet.
   R_{f} (E/H 1/1) = 0,3 MS (FAB) = 512 (M + 1)
e) 4-[3-(N-Phthaloylglycyl-N-methylamino)-2,6-dichlorobenzyloxy]-benzthiazol
   Zur Titelverbindung gemäß Beispiel 1d) (1,8 g, 3,5 mmol) in trockenem DMF (50 ml) wurde unter Argon 50 % NaH (170 mg, 3,5 mmol einer 60 % Suspension in Mineralöl) bei 0°C zugegeben. Nach 30 min wurde Methyljodid (500 mg, 3,5 mmol) zugespritzt. Nach weiteren 2h bei 0°C wurde auf H₂O (200 ml) gegossen und 3 mal mit Ethylacetat extrahiert. Die vereinten organischen Extrakte wurden 3 mal mit H₂O gewaschen, getrocknet (MgSO₄) und eingeengt. Chromatographie an SiO₂ mit E/H1/1 als Eluens lieferte die Titelverbindung.
   R_{f} (E/H 1/1) = 0,2 MS (ESI) = 526 (M+1)
f) 4-[3-(N-Glycyl-N-methylamino)-benzyloxyl-benzthiazol
   Zur Titelverbindung gemäß Beispiel 1e) (750 mg, 1,4 mmol) in Ethanol (20 ml) wurde bei RT Hydrazinhydrat (0,3 ml) zugegeben. Nach 1h wurde eingeengt und Dichlormethan (20 ml) zugegeben. Der entstandene Niederschlag wurde abgesaugt und das Filtrat eingeengt, wobei die Titelverbindung anfiel, die roh für den nächsten Reaktionsschritt verwendet wurde.
   MS(ESI) = 396 (M+1)
g) 4-[3-(N-(3-Methoxycinnamoylglycyl)-N-methylamino)-2,6-dichlorobenzyloxy]-benzthiazol
   Zu 3- Methoxyzimtsäure (130 mg, 0,75mmol) in DMF (20 ml) wurden Dicyclohexylcarbodiimid (DCC) (170 mg, 0,83 mmol) und Hydroxybenzotriazol (HOBt) (150 mg, 1,13 mmol) bei RT zugegeben. Nach 30 min wurde die Titelverbindung gemäß Beispiel 1f) (300 mg, 0,75 mmol) in DMF (2 ml) zugegeben. Nach 18h bei RT wurde mit Ethylacetat (100 ml) verdünnt und je 2 mal mit 100 ml gesättiger Na₂CO₃-, NaCl-Lösung und H₂O gewaschen, getrocknet (MgSO₄) und eingeengt. Chromatographie an SiO₂ mit E/H 3/1 als Eluens lieferte die Titelverbindung des Beispiels 1.
   R_{f} (E/H 1/1) = 0,2 MS (FAB) = 556 (M+1)

### Beispiel 2

### 6-[3-(N-(3-Methoxycinnamoylglycyl)-N-methylamino)-2,6-dichlorobenzyloxy]-benzthiazol

Die Titelverbindung wurde analog Beispiel 1 erhalten.
R_{f} (E/H 1/1) = 0,2 MS (FAB) = 556 (M+1)

### Beispiel 3

### 4-[3-(N-4-Trifluormethylcinnamoylglycyl)-N-methylamino)-2,6-dlchlorobenzyloxy]-2-methylbenzthiazol

a) 2-Methoxy-acetylanilin
   Zu 2-Methoxyanilin (15 g, 120 mmol) und Triethylamin (12,3 g. 120 mmol) in Dichlormethan (100 ml) wurden unter Argon bei Temperaturen zwischen 0°C und 10°C Acetylchlorid (9,6 g, 120 mmol) zugegeben. Nach 2h wurde Dichlormethan (100 ml) zugegeben, je 1 mal (100 ml) mit gesättigter NaHCO₃und 5 % NaHSO₄-Lösung gewaschen, getrocknet (CaCl₂) und eingeengt, wobei die Titelverbindung als amorphes Pulver anfiel.
   R_{f} (E/H 1/1) = 0,2 MS (ESI) = 166 (M+1)
b) 2-Methoxy-thioacetylanilin
   Zur Titelverbindung gemäß Beispiel 3a) (17,5 g, 106 mmol) in Butylacetat (100 ml) wurde P₂S₁₀ (11,7 g, 53 mmol) gegeben und das Gemisch 5h zum Sieden erhitzt. Nach Abkühlen auf RT wurde je 1 mal mit H₂O (100 ml) und gesättigter NaHCO₃ Lösung (100 ml) und nochmals mit H₂O (100 ml) gewaschen, getrocknet (MgSO₄) und eingeengt, wobei die Titelverbindung als Öl anfiel
   R_{f} (E/H 1/1) = 0,35 MS (ESI) = 182 (M+1)
c) 4-Methoxy-2-methylbenzthiazol
   Ein Gemisch aus der Titelverbindung gemäß Beipsiel 3b) (19 g, 105 mmol), 10 % NaOH (300 ml) und Ethanol (40 ml) wurde bei 80-90°C langsam zu einer Lösung aus Kaliumhexacyanoferrat(III) (138 g, 419 mmol) in H₂O (350 ml) gegeben. Nach Beendigung der Zugabe wurde die Temperatur noch 4h bei 80-90°C gehalten und dann ließ man das Gemisch auf RT abkühlen. Nun wurde 3 mal mit Ethylacetat (3 x 300 ml) extrahiert, die vereinten organischen Extrakte getrocknet (MgSO₄) und eingeengt. Chromatographie an SiO₂ mit E/H 1/1 als Eluens lieferte die Titelverbindung als amorphes Pulver.
   R_{f} (E/H 1/1) = 0,3 MS (DCI) = 180 (M+1)
d) 4-Hydroxy-2-methylbenzthiazol
   Die Titelverbindung gemäß Beispiel 3c) (16.4 g, 91,6 mmol), Jodwasserstoffsäure (57 %ig, 70 ml), Essigsäure (15 ml) und roter Phosphor (4,2 g) wurden 10h unter Rückfluß gekocht. Nach Abkühlen auf RT wurde H₂O (200 ml) zugegeben, der pH wurde mit 2n NaOH auf 5 gestellt und 3 mal mit Ethylacetat (je 200 ml) extrahiert. Die vereinten organischen Extrakte wurden getrocknet (MgSO₄) und eingeengt, wobei die Titelverbindung als amorphes Pulver anfiel.
   R_{f} (E/H 1/1) = 0,4 MS (DCI) = 166 (M+1)
e) trans-4-Trifluormethylzimtsäurechlorid
   Zu 4-Trifluormethyl-E-zimtsäure (1 g, 4,6 mmol) und Pyridin (375 µl, 4,6 mmol) in trockenem CH₂Cl₂ (11 ml) wurde bei 0°C Thionylchlorid (335 µl, 4,6 mmol) zugegeben. Anschließend wurde 1 h ohne Kühlung gerührt, wieder auf 0°C gekühlt und unter Feuchtigkeitsausschluß filtriert.
   Das Filtrat (10 ml) enthielt die Titelverbindung und wurde in Aliquoten für den nächsten Reaktionsschritt verwendet.
f) 4-[3-(N-Glycyl-N-methylamlno)-2,6-dichlorobenzyloxy]-2-methylbenzthiazol
   Die Titelverbindung wurde analog Beispiel 1 b)-f) erhalten.
   R_{f} (Aceton/H₂O 10/1) = 0,3 MS (ESI) = 410 (M+1)
g) 4-[3-(N-4-Trifluormethylcinnamoylglycyl)-N-methylamino)-2,6-dichlorobenzyloxyl-2-methylbenzthiazol
   Zur Titelverbindung gemäß 3f) (245 mg, 0,6 mmol) in Dichlormethan (5 ml) wurde ein Aliquot der Lösung der Titelverbindung gemäß Beispiel 3e) (2 ml, 1,5 eq, 0,9 mmol) bei RT zugegeben. Nach 18h wurde gesättigte Na₂CO₃-Lösung (10 ml) zugegeben und 3 mal mit Dichlormethan (3 x 20 ml) extrahiert. Die organischen Phasen wurden getrocknet (CaCl₂) und eingeengt. Chromatographie an SiO₂ mit Ethylacetat als Eluens lieferte die Titelverbindung Beispiel 3 als amorphes Pulver.
   R_{f} (E) = 0,45 MS (FAB) = 608 (M+1)
h) Alternative Synthese der Titelverbindung gemäß Beispiel 3
   Ein Gemisch bestehend aus der Titelverbindung gemäß Beispiel 3f) (200 mg, 0,49 mmol) 4-Trifluormethylzimtsäure (106 mg, 0,49 mmol) O-[Cyan(ethoxycarbonyl)-methylenaminol-1,1,3,3-tetramethyluroniumtetrafluoroborat (Totu) (160 mg, 0,49 mmol), Triethylamin (49 mg, 0,49 mmol) in NMP (10 ml) wurde 1h bei RT gerührt. Nun wurde mit Ethylacetat (100 ml) verdünnt, je 2 mal mit gesättigter Na₂CO₃ und 1 mal mit H₂O (je 100 ml) gewaschen, getrocknet (MgSO₄) und eingeengt. Chromatographie an SiO₂ mit Ethylacetat als Eluens lieferte die Titelverbindung gemäß Beispiel 3.

Die Beispiele 4-39 können analog Beispiel 1 und 3 erhalten werden (Tab. 1).

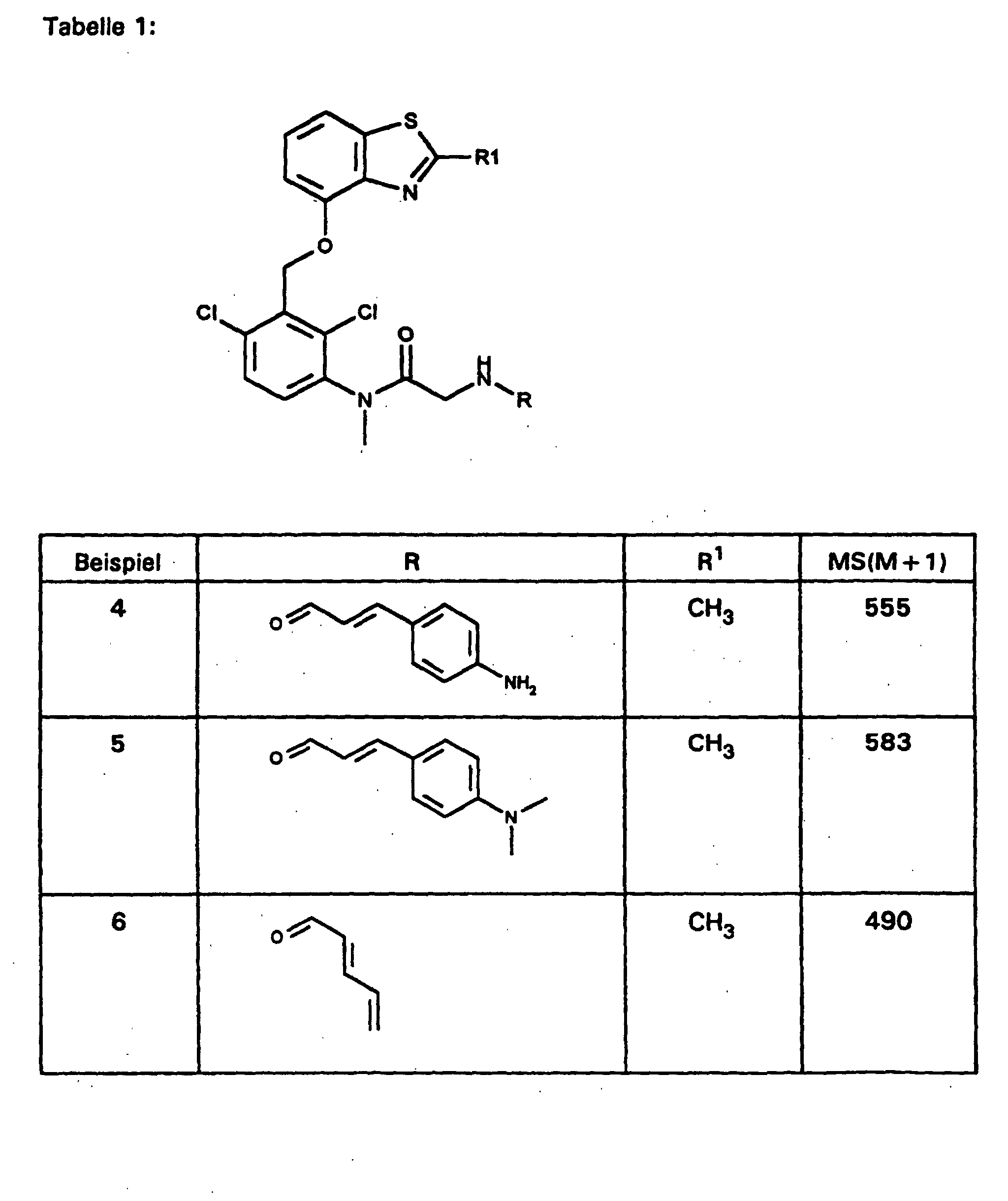

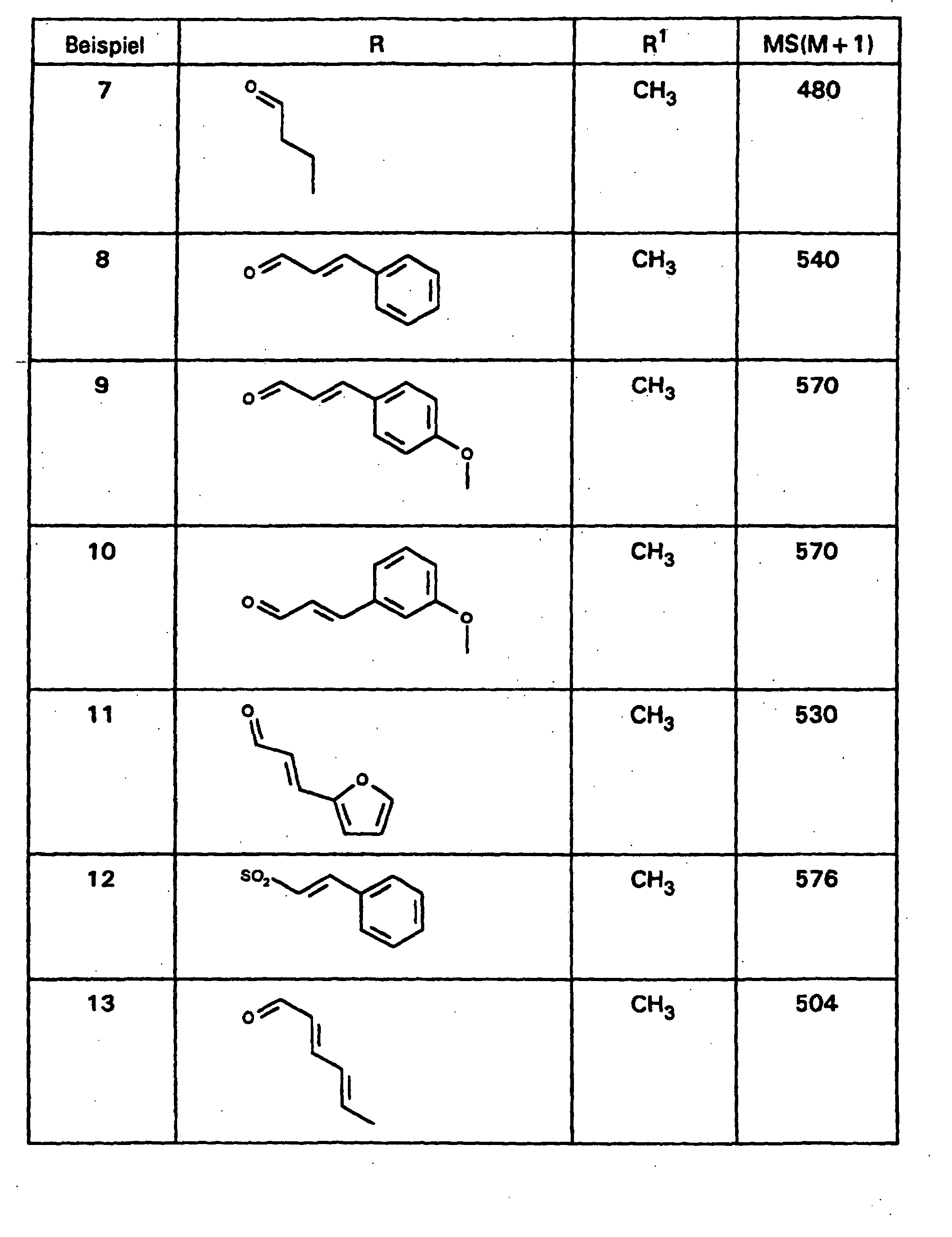

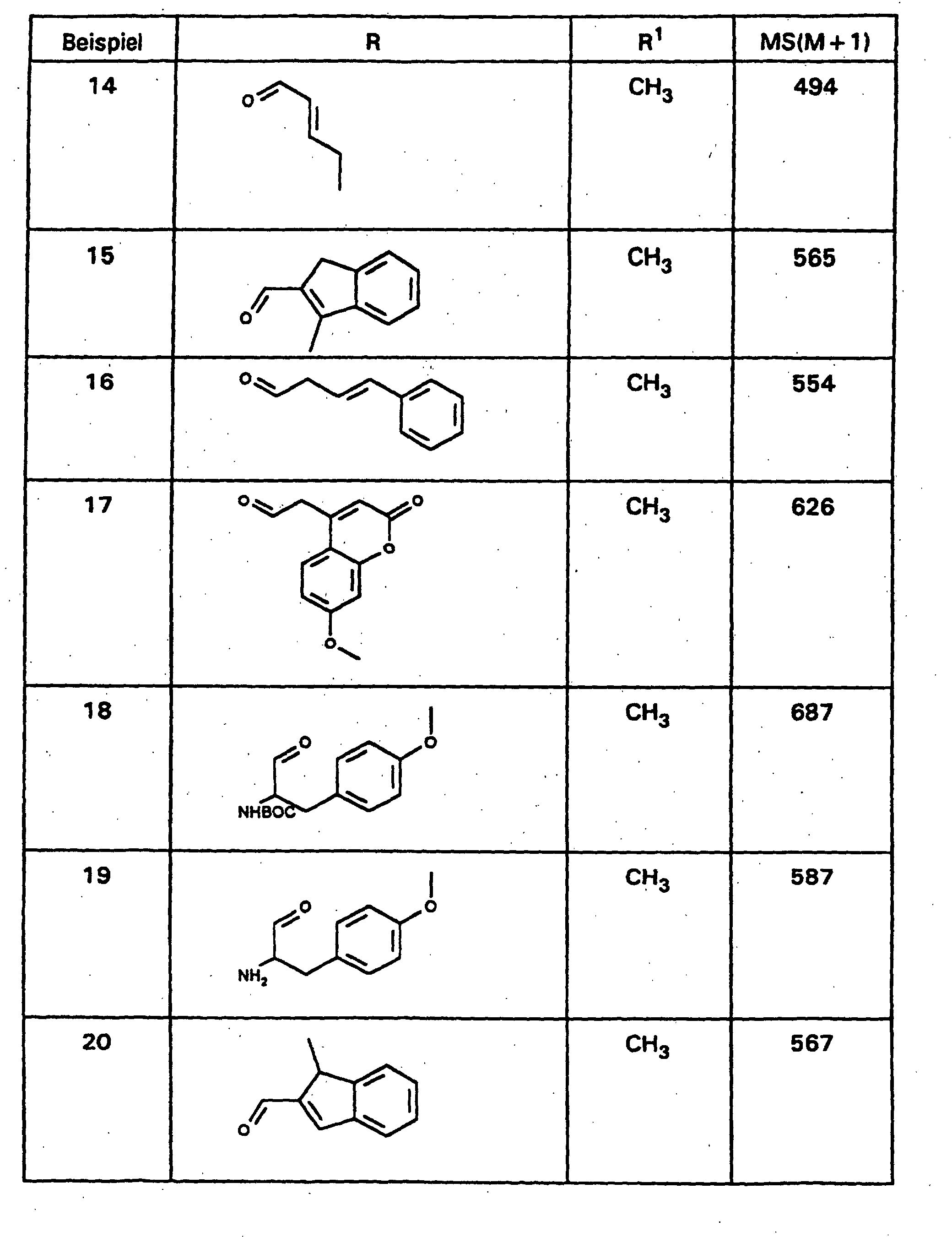

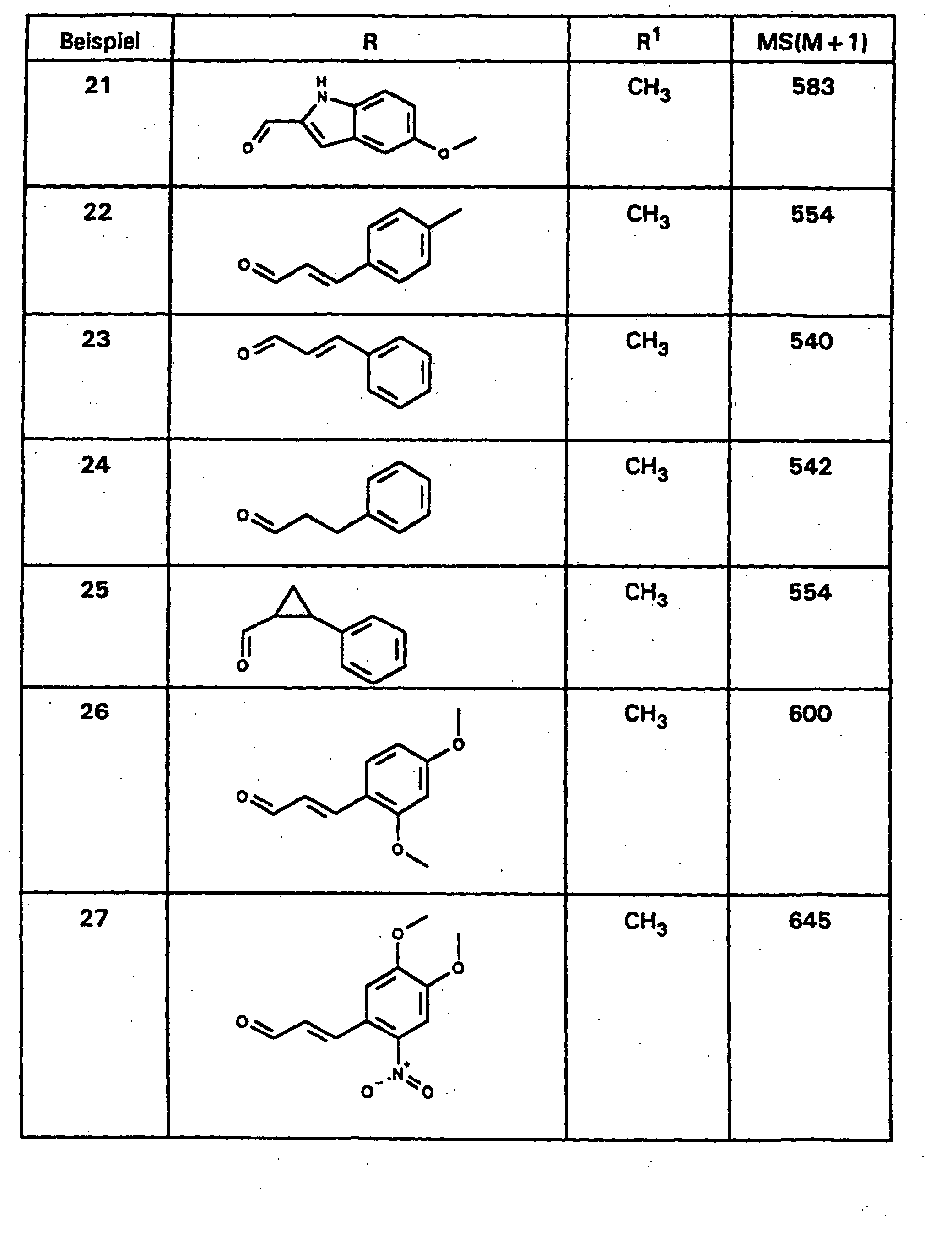

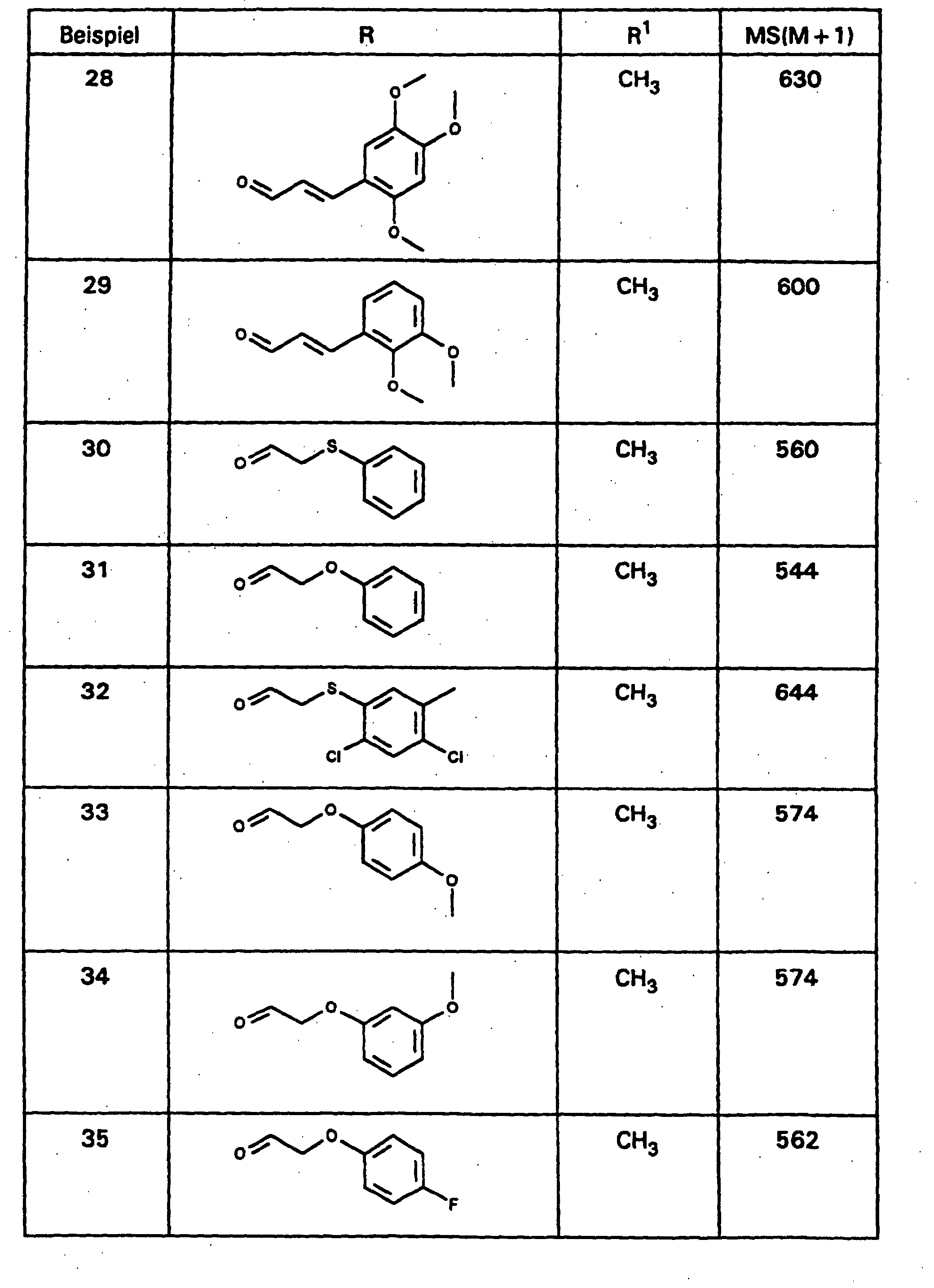

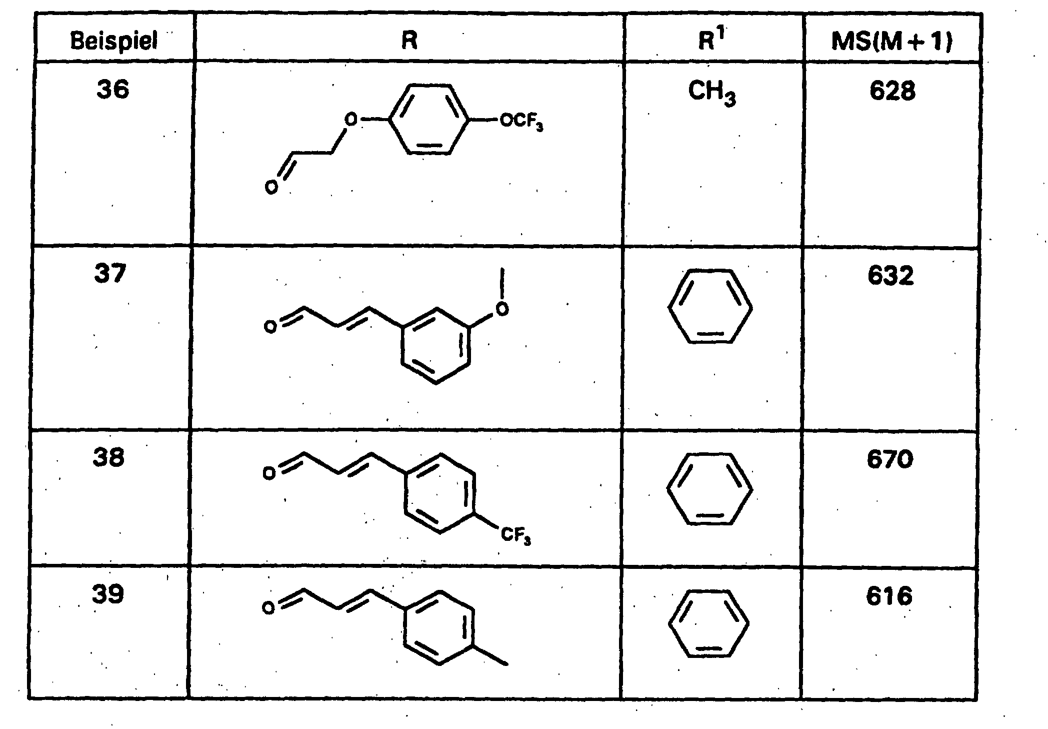

### Beispiel 40

### 4-[3-(N-4-Methoxybenzylureidoacetyl)-N-methylamino)-2,6-dichlorobenzyloxy]-2-methylbenzthiazol

Die Titelverbindung gemäß Beispiel 3f (200 mg, 0,49 mmol), Diisopropylethylamin (63 mg, 0,49 mmol) und N,N-Carbonyldiimidazol (80 mg, 0,49mmol) in NMP (10 ml) wurden 4h bei RT gerührt. Anschließend wurde 4-Methoxybenzylamin (60 mg, 0,49 mmol) zugesetzt. Nach weiteren 18h wurde Ethylacetat (100 ml) zugegeben und je 1 mal mit je 50 ml gesättigter Na₂CO₃-, 5 % NaHSO₄-Lösung und H₂O gewaschen, getrocknet (MgSO₄) und eingeengt. Chromatographie an SiO₂ mit Ethylacetat als Eluens lieferte die Titelverbindung als amorphes Pulver.
R_{f} (E) = 0,15 MS (FAB) = 573 (M+1)

Die Verbindungen der Beispiele 41 und 42 wurden analog Beispiel 40 erhalten.

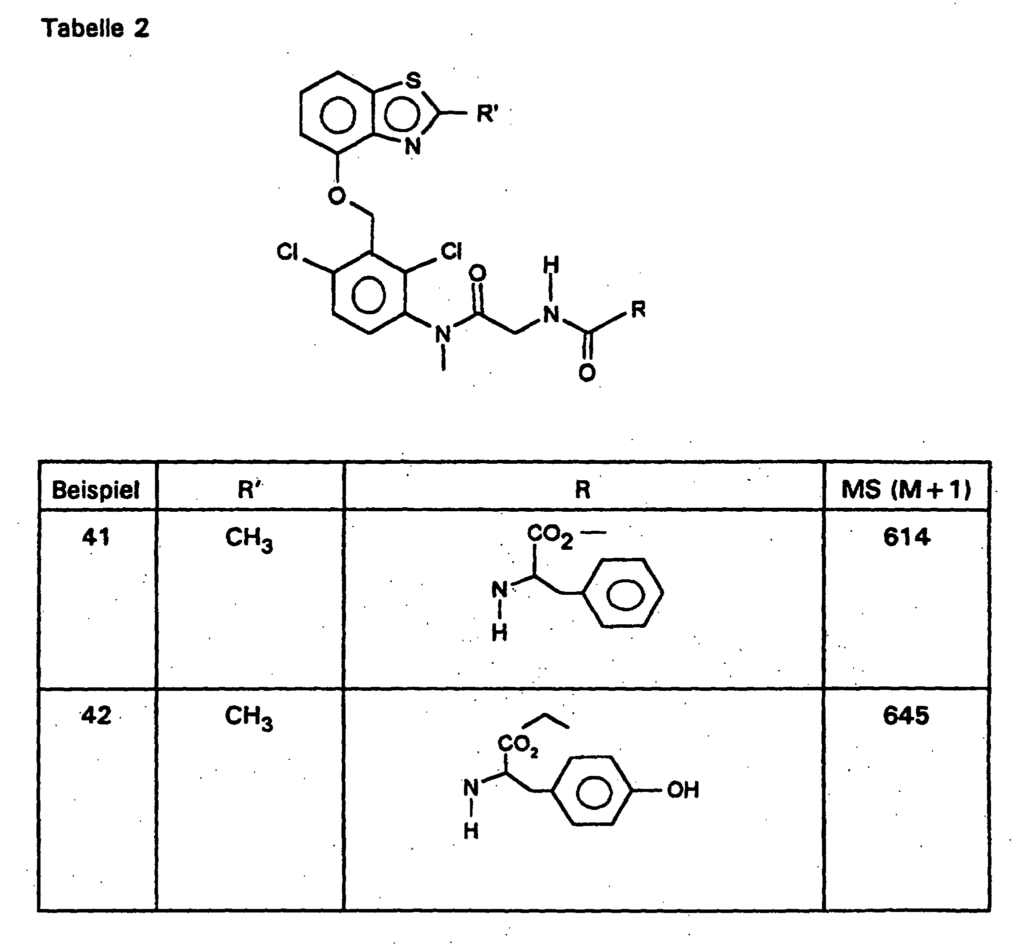

### Beispiel 43

### 4-[3-(N-Benzyloxycarbonylaminoacetyl-N-methylamino)-2,6-dichlorobenzyloxy]-2-methylthiazol

a) Zur Titelverbindung gemäß Beispiel 3f) (200 mg, 0,49 mmol) und Diisopropylethylamin (63 mg, 0,49 mmol) in NMP (10 ml) wurde bei RT N-Benzyloxycarbonyl-succinimid (112 mg, 0,49 mmol) zugegeben. Nach 18 h wurde auf H₂O (50 ml) gegossen und 3 mal mit Ethylacetat (je 50 ml) extrahiert. Die vereinten organischen Extrakte wurden getrocknet (MgSO₄) und eingeengt. Chromatographie an SiO₂ mit Ethylacetat als Lösungsmittel lieferte die Titelverbindung gemäß Beispiel 43 als amorphes Pulver
   R_{f} (E) = 0,5 MS (FAB) = 544 (M+1)
b) Alternative Synthese der Titelverbindung gemäß Beispiel 43
   Benzylalkohol (52 mg, 0,49 mmol), Diisopropylethylamin (63 mg, 0,49 mmol), N,N-Carbodiimidazol (80 mg, 0,49 mmol) und eine Spatelspitze DMAP wurden bei RT 8h gerührt. Anschließend wurde die Titelverbindung gemäß Beispiel 3f) (200 mg, 0,49 mmol) zugegeben. Nach 18h wurde auf H₂O (50 ml) gegossen und 3 mal mit Ethylacetat (je 50 ml) extrahiert. Die vereinten organischen Extrakte wurden getrocknet (MgSO₄) und eingeengt. Chromatographie an SiO₂ mit Ethylacetat als Eluens lieferte die Titelverbindung gemäß Beispiel 43.

### Beispiel 44

### 4-[3-N(4-Trans-trifluormethylcinnamoylglycyl)-N-methylamino)-6-chloro-2-methoxy-benzyloxy]-2-methylbenzthiazol

a) 2,6-Chlor,methoxy-3-nitrotoluol und 2,6-Dimethoxy-3-nitrotoluol
Zu Natriumhydrid (5,8 g einer 60 % Suspension in Mineralöl) in DMF (200 ml) wurde Methanol (5,8 ml, 0,145 Mol) bei 0°C zugegeben. Nach 30 min wurde 2,6-Dichlor-3-nitrotoluol (30 g, 0,145 Mol) in DMF zugespritzt, wobei die Temperatur auf ~ 20°C anstieg. Anschließend wurde 1,5h ohne Kühlung gerührt, dann Eis (∼ 300 g) zugegeben und 3 mal mit Ethylacetat (3 x 800 ml) extrahiert. Die Extrakte wurden getrocknet (MgSO₄) und im Vakuum eingeengt. Chromatographie an SiO₂ mit E/H 1/2 als Eluens liefert die drei Titelverbindungen als Öle.

| | | |
|---|---|---|
| 1. | Isomeres (2-Methoxy, 6-Chloro) | R_{f} (E/H 1/2) = 0,4 |
| | 2,6-Dimethoxy-3-nitrotoluol | " = 0,3 |
| | | |
| 2. | Isomeres (2-Chloro, 6-Methoxy) | " = 0,25 |

MS (DCI) = 202 (M + 1) für die beiden Chlor, Methoxy Isomeren
MS (DCI) = 198 (M + 1) für das Dimethoxy-Produkt.
b) 2-Methoxy-6-chloro-3-nitrobenzylbromid
1. Isomeres aus der Titelverbindung gemäß Beispiel 44a (8 g, 40 mmol) wurde analog der Titelverbindung gemäß Beispiel 1a umgesetzt.
   R_{f} (E/H 1/2) = 0,35 MS(DCI) = 280 (M+1)

c) Die Titelverbindung gemäß Beispiel 44 wurde analog den Beispielen 1 und 3 erhalten.
R_{f} (E) = 0,4 MS (FAB) = 604 (M + 1)

Die Verbindungen der Beispiele 45 und 46 können analog Beispiel 44 erhalten werden.

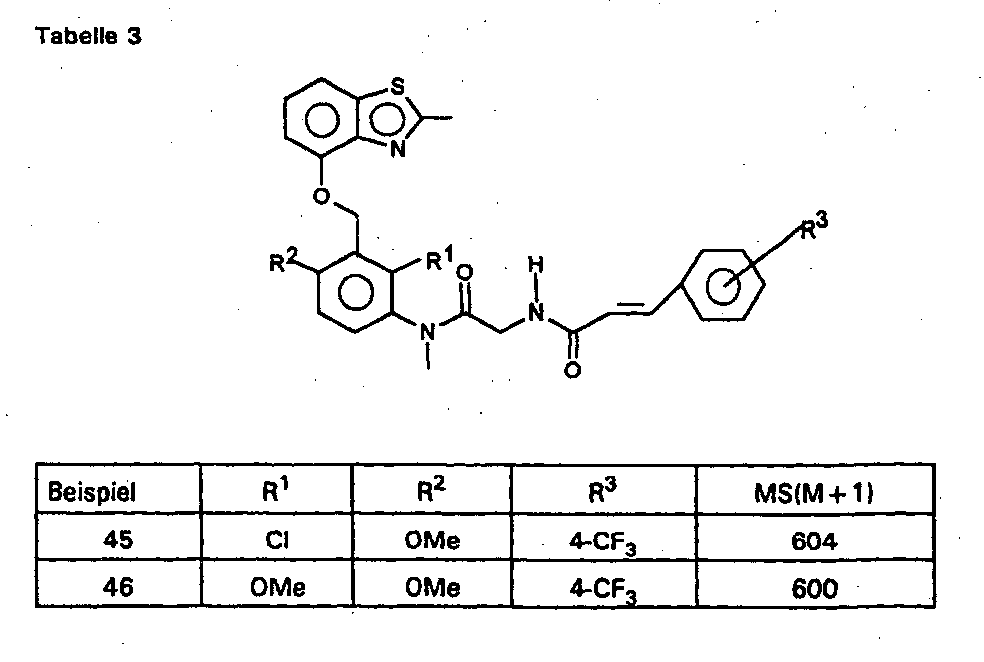

## Patentansprüche

1. Verbindungen der Formel I 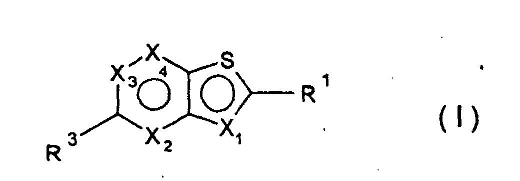 worin die Symbole folgende Bedeutungen haben:
a) einer der Reste X₁, X₂ oder X₃
steht für C-O-R² und die jeweils anderen X₁, X₂, X₃ und X₄ sind dann gleich oder verschieden
1. N
2. CR¹;
b) R¹ und R³ gleich oder verschieden
1. H
2. Halogen
3. (C₁-C₆)-Alkyl
4. O-R⁶
5. S-R⁶
6. NHR⁶
7. (C₆-C₁₂)-Aryl
8. (C₆-C₁₂)-Aryl-(C₁-C₃)-Alkyl
9. C(O)-OR⁶
10. C(O)-H
11. (C₂-C₅)-Alkenyl
12. NO₂
13. SO₃R⁷
14. CN
15. C(O)-NHR⁸
wobei 3., 7., 8., 11. gegebenenfalls mit einer oder mehreren Gruppen wie C(O)-(O)ₒ-(C₁-C₅)-Alkyl, OR⁶, SR⁷, NO₂, CN, NHR⁸, Halogen substituiert sein können;
c) R² ein Rest der Formel (II) 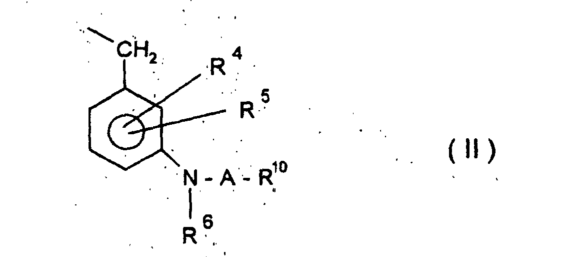 bedeutet;
d) R⁴ und R⁵ gleich oder verschieden
1. H
2. Halogen
3. OR⁶
4. SR⁶
5. CN
6. (C₁-C₅)-Alkyl bedeuten;
e) R⁶, R⁷ und R⁸ gleich oder verschieden
1. H
2. (C₁-C₅)-Alkyl
3. (C₃-C₅)-Alkenyl
4. (C₆-C₁₂)-Aryl-(C₁-C₃)-Alkyl;
5. (C₃-C₁₀)-Cycloalkyl,
6. (C₃-C₁₀)-Cycloalkyl-(C₁-C₃)-Alkyl;
7. C(O)-(O)ₒ-(C₁-C₅)-Alkyl,
8. C(O)-(NH)ₒ-(C₁-C₅)-Alkyl;
f) A eine Aminocarbonsäure ausgewählt aus der Gruppe Methionin, Alanin, Phenylalanin, 2-Chlorphenylalanin, 3-Chlorphenylalanin, 4-Chlorphenylalanin, 2-Fluorphenylalanin, 3-Fluorphenylalanin, 4-Fluorphenylalanin, Tyrosin, O-Methyltyrosin, β-(2-Thienyl)alanin, Glycin, Cyclohexylalanin, Leucin, Isoleucin, Valin, Norleucin, Phenylglycin, Serin, Cystein, Aminopropionsäure oder Aminobuttersäure;
g) R⁹
1. H
2. C(O)-(NH)ₒ-(C₁-C₅)-Alkyl;
2. C(O)-(O)ₒ-(C₁-C₃)-Alkyl-(C₆-C₁₀)Aryl;
h) R¹⁰
1. -C(O)-D-E
2. -C(S)-D-E
3. -SO₂-D-E
4. Wasserstoff;
i) D
1. (C₂-C₅)-Alkendiyl
2. (C₁-C₈)-Alkandiyl
3. -(CH₂)ₙ-Yₒ-(CH₂)ₘ-
4. (C₃-C₁₀)-Cycloalkandiyl
5. (C₃-C₁₀)-Cycloalkyl-(C₁-C₃)-alkandiyl
6. (C₃-C₁₀)-Cycloalkendiyl
7. (C₃-C₁₀)-Cycloalkenyl-(C₁-C₃)-alkandiyl
wobei 1.-7. gegebenenfalls mit einer oder mehreren Gruppen wie z.B. OR⁶, NO₂, CN, CO₂R⁷, NR⁸R⁹, SO₂R⁶, SO₂NR⁸R⁹, SO₃R⁷ oder C(O)-NR⁸R⁹ substituiert sein kann;
j) E
1. H
2. (C₆-C₁₀)-Aryl,
3. (C₁-C₉)-Heteroaryl,
wobei 2. und 3. gegebenenfalls mit einer oder mehreren Gruppen substituiert sein können wie z.B. NR⁸R⁹, CN, CO₂R⁶, SO₃R⁷, NO₂, SO₂NR⁸R⁹, SO₂R⁶, O-(C₁-C₅)-Alkyl, S-(C₁-C₅)-Alkyl, (C₁-C₅)-Alkyl, (C₂-C₅)-Alkenyl, wobei O-(C₁-C₅)-Alkyl und (C₁-C₅)-Alkyl gegebenenfalls teilweise oder vollständig mit Halogen substituiert sein können;
k) Y
1. O
2. S
3. NR⁸;
l) n und m gleich oder verschieden eine Zahl 0-6;
m) o 0, 1;
sowie deren physiologisch verträglichen Salze.

2. Verbindungen der Formel (I) gemäß Anspruch 1, worin die Symbole folgende Bedeutung haben:
a) R¹ und R³ gleich oder'verschieden
1. H
2. (C₁-C₆)-Alkyl
3. O-R⁶
4. S-R⁶
5. NHR⁶
6. (C₂-C₅)-Alkenyl
7. C(O)-OR⁶
8. C(O)-H
9. NO₂
10. CN
11. C(O)-NHR⁸
wobei 2. und 6. gegebenenfalls mit einem oder mehreren Gruppen wie z.B. Halogen, CO₂R⁶, NHR⁸ substituiert sein können;
b) R⁶, R⁷ und R⁸ gleich oder verschieden
1. H
2. (C₁-C₅)-Alkyl
3. (C₆-C₁₀)-Aryl-(C₁-C₃)-Alkyl bedeuten.

3. Verbindungen der Formel (I) gemäß Anspruch 1 oder 2, worin
a) R¹ und R³ gleich oder verschieden
1. H
2. (C₁-C₄)-Alkyl
3. NH-(C₁-C₅)Alkyl
4. O-(C₁-C₅)Alkyl
5. S-(C₁-C₅)-Alkyl
6. C(O)-H
7. CO₂R⁶
8. (C₂-C₃)-Alkenyl
wobei 2.-5. und 8. mit einem oder mehreren Resten wie z.B. Halogen, CO₂R⁶, NHR⁸ substituiert sein können, bedeuten;
b) A Leucin, Isoleucin, Valin, Alanin, Methionin, Glycin, Serin, Aminopropionsäure, Aminobuttersäure bedeutet.

4. Verfahren zur Herstellung von Verbindungen der Formel (I) gemäß den Ansprüchen 1 bis 3, **dadurch gekennzeichnet, daß** man
a₁)
1. eine Verbindung der Formel (III) 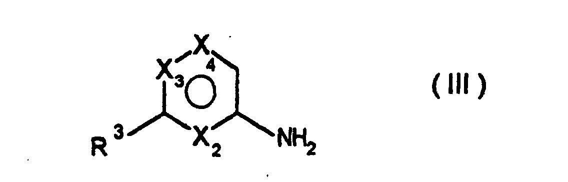 worin R³, X₂, X3 und X₄ wie oben in Formel I definiert sind, zuerst mit aktivierten Carbonsäurederivaten unter Verwendung einer Hilfsbase bei Temperaturen zwischen 0-20°C acyliert,
2. die so erhaltene Verbindung der Formel (IV) 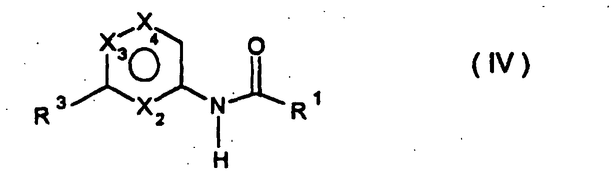 mit Laweson's Reagenz in Butylacetat oder anderen inerten hochsiedenden Lösungsmitteln zum Sieden erhitzt und so eine Verbindung der Formel (V) erhält, 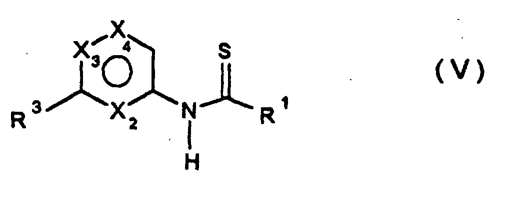 worin R¹, R³, X₂, X3 und X₄ in Formeln IV und V wie oben in Formel I definiert sind, für X₂ oder X₃ = C-O-R² steht R² für R²' = H oder (C₁-C₅)-Alkyl,
3. die so erhaltene Verbindung (V) durch radikalische Cyclisierung mit radikalerzeugenden Reagenzien, bevorzugt K₃Fe(CN)₆ oder Br₂ in inerten Lösungsmitteln bei Temperaturen zwischen 80-110°C umsetzt und dabei eine Verbindung der Formel (VI) erhält 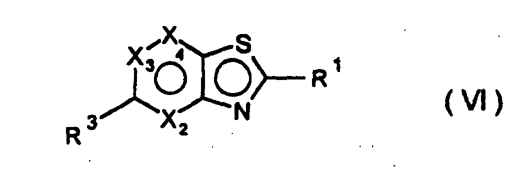 worin die Reste R¹, R³, X₂, X₃ und X₄ wie oben in Formel I definiert sind, für X₂ oder X₃ = C-O-R² steht R² für R^{2'} = H oder (C₁-C₅)-Alkyl,
4. eine Verbindung der Formel (VI), worin X₂ oder X₃ C-O-R² bedeutet und wie unter 3. definiert ist, durch etherspaltende Reagenzien in inerten Lösungsmitteln oder ohne Lösungsmittel bei Temperaturen zwischen 0°C und dem Siedepunkt, zu Verbindungen der Formel (VI) umsetzt, worin X² oder X³ COR² bedeutet und R² = R²' = Wasserstoff ist;
oder
a₂)
1. eine Verbindung der Formel (VII) 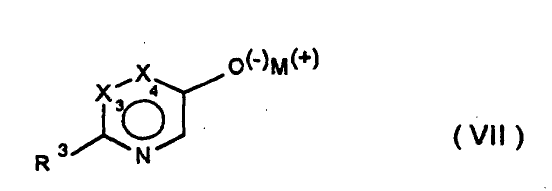 worin R³, X₃, X₄ wie oben in Formel I definiert sind und M für Kalium, Natrium oder Cäsium steht,
durch sukzessive Behandlung mit CO₂ und anschließend NH₃ unter erhöhtem Druck und Temperaturen, bevorzugt 100 atm und 200°C, in eine Verbindung der Formel (VIII) umwandelt, 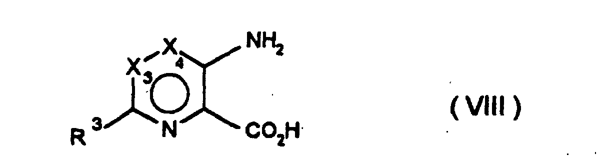 worin R³, X₃, X₄ wie oben in Formel I definiert sind,
2. eine Verbindung der Formel (VIII) durch Diazotierung und anschließender Behandlung mit HS-CHR¹-CO₂H in eine Verbindung der Formel (IX) umwandelt, 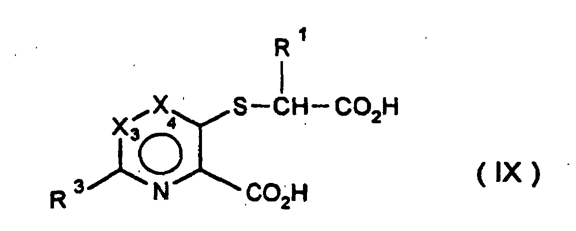 worin R¹, R³, X₃, X₄ wie oben in Formel I definiert sind,
3. eine Verbindung der Formel (IX) durch Cyclisierung unter gleichzeitiger Decarboxylierung und Wasserabspaltung in inerten Lösungsmitteln oder ohne Lösungsmittel in eine Verbindung der Formel (X) überführt, 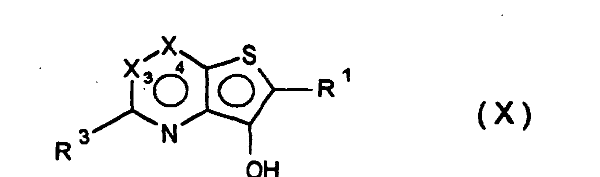 worin R¹, R³, X₃, X₄ wie oben in Formel (I) definiert sind;
b) eine Verbindung der Formeln (VI) oder (X) 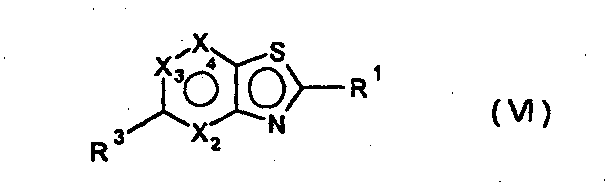 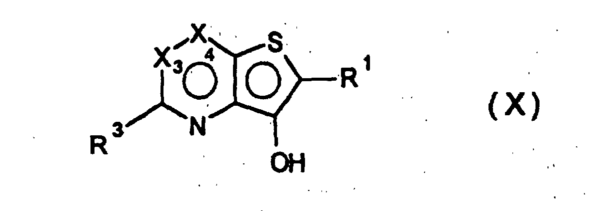 worin X₂, X₃, X₄, R¹ und R³ wie oben in Formel (I) definiert sind, im Falle von Verbindungen der Formel (VI) X₂ oder X₃ = C-O-H bedeutet, mit Cs₂CO₃ oder K₂CO₃ in einem inerten Lösungsmittel deprotoniert und bei Raumtemperatur mit einer Verbindung der Formel (XI) 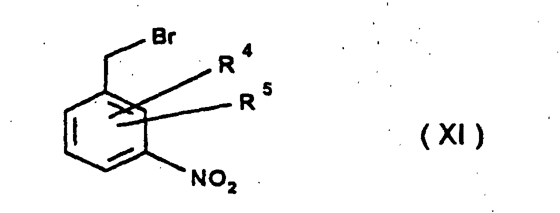 worin R⁴ und R⁵ wie oben in Formel (II) definiert sind, umsetzt;
c) die so erhaltenen Verbindungen der Formeln (XII) oder (XII') 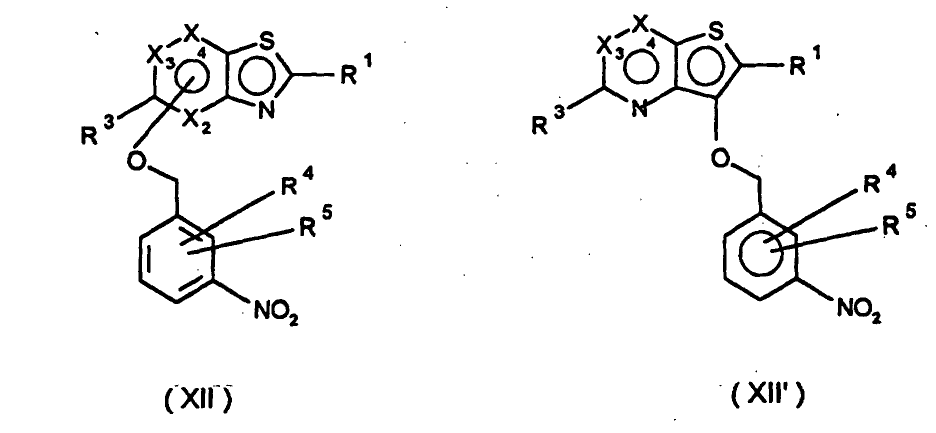 worin R¹, R³, R⁴, R⁵, X₂, X₃, X₄ wie oben in Formel (I) und (II) definiert sind, mit Hilfe von Übergangsmetallhalogeniden zu einer Verbindung der Formeln (XIII) oder (XIII') reduziert, 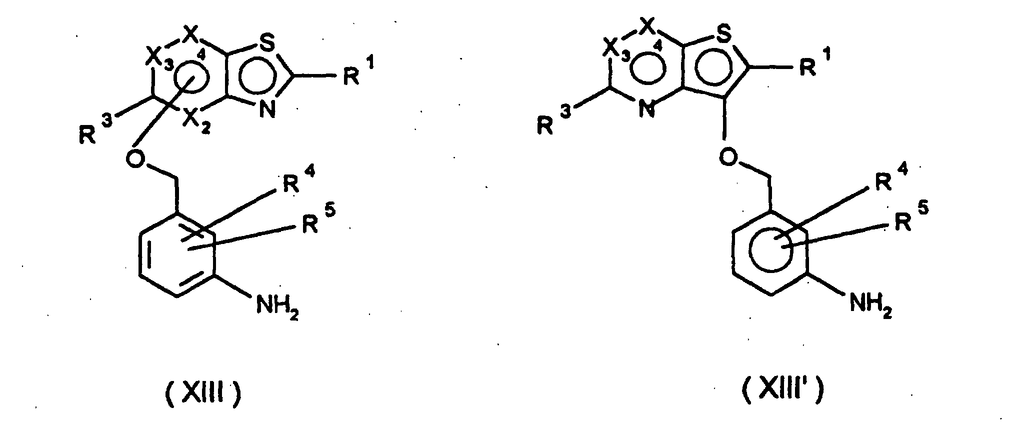 worin R¹, R³, R⁴, R⁵, X₂, X₃ und X₄ wie oben in Formeln (I) und (II) definiert sind;
d) eine Verbindung der Formeln (XIII) oder (XIII') mit aktivierten, geeignet geschützten Aminocarbonsäurederivaten von A (A-Prot), in inerten Lösungsmitteln, gegebenenfalls durch Zugabe von DMAP, umsetzt und so eine Verbindung der Formeln (XIV) oder (XIV') erhält, 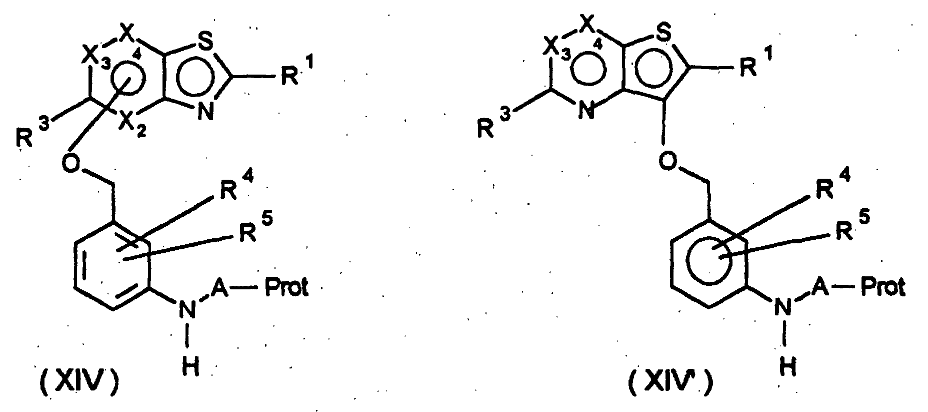 worin A, R¹, R³, R⁴, R⁵, X₂, X₃ und X₄ wie oben in Formeln (I) und (II) definiert sind, und
Prot für eine Aminoschutzgruppe steht, wobei beide Protonen der Aminoschutzgruppe geschützt sind;
e) eine Verbindung der Formeln (XIV) oder (XIV') nach erfolgter Einwirkung von Alkalihydriden, Alkalicarbonaten oder Alkoholaten in inerten Lösungsmitteln, gefolgt von einer Behandlung mit R⁶X, wobei R⁶ wie oben in Formel (I), ausgenommen R⁶ = H, definiert ist und X eine Abgangsgruppe bedeutet, umsetzt, wobei man eine Verbindung der Formeln (XV) oder (XV') erhält, 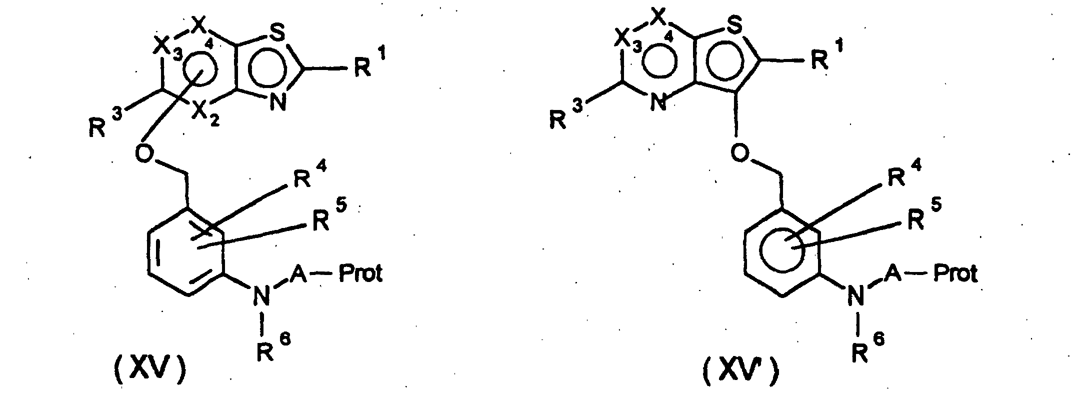 worin A, R¹, R³, R⁴, R⁵, R⁶, X₂, X₃ und X₄ wie oben in Formel (I) und (II) und Prot wie oben in Formel (XIV) definiert sind;
f) zum Entfernen der Schutzgruppe (Prot.) von der Verbindung der Formeln (XV) oder (XV'), im Falle der Phthaloylgruppe bevorzugt mit Hydrazin in Alkoholen als Lösungsmittel bei Temperaturen zwischen Raumtemperatur und dem Siedepunkt umsetzt, wobei man eine Verbindung der Formeln (XVI) oder (XVI') erhält, 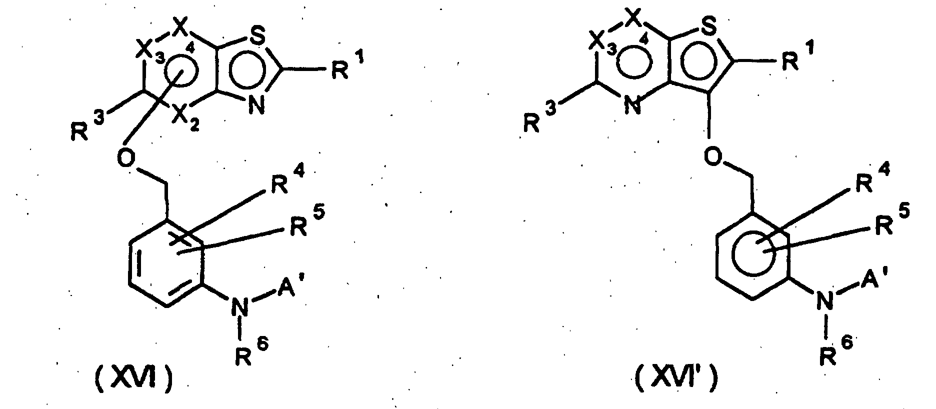 worin R¹, R³, R⁴, R⁵, R⁶, X₂, X₃ und X₄ wie oben in Formeln (I) und (II) und Prot wie oben in Formel (XIV) definiert sind und A' ein Rest der Aminosäure A ist;
g₁) eine Verbindung der Formeln (XVI) oder (XVI') mit aktivierten Säurederivaten der Formeln (XVII), (XVIII) oder (XIX)
E - D- C(O) - OH (XVII)
E - D - C(S) - OH (XVIII)
E - D - SO₂ - OH (XIX)
worin D und E wie oben in Formel (II) definiert sind,
umsetzt, oder
g₂) eine Verbindung der Formeln (XVI) oder (XVI') mit einem Amin oder einem Alkohol der Formel (XX)
E - D - Z (XX)
worin E und D wie oben definiert sind und Z OH oder NH₂ bedeuten, wobei man zunächst jedoch die Verbindungen der Formeln (XVI), (XVI') oder (XX) mit einer doppelt aktivierten Carbonylverbindung zur Bildung der Harnstoff- bzw. Urethangruppe, z.B. mit Carbodiimiden, Phosgen oder Chlorkohlensäureestern reagieren läßt in inerten Lösungsmitteln umsetzt oder
g₃) eine Verbindung der Formeln (XVI) oder (XVI') mit einem entsprechenden Isocyanat oder Isothiocyanat in inerten Lösungsmitteln umsetzt, und
h) die erhaltene Verbindung der Formel (I) gegebenenfalls nach bekannten Methoden in ihre physiologisch verträglichen Salze überführt.

5. Verwendung der Verbindungen der Formel (I) gemäß den Ansprüchen 1 bis 3 als Heilmittel.

6. Heilmittel enthaltend mindestens eine Verbindung der Formel (I) gemäß den Ansprüchen 1 bis 4.

## Claims

1. A compound of the formula I 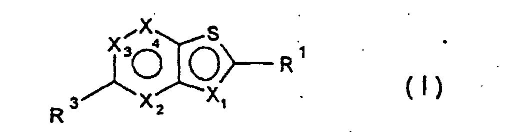 in which the symbols have the following meanings:
a) one of the radicals X₁, X₂ or X₃
is C-O-R^{2,} and the other X₁, X₂, and X₃, in each case, and X₄, are then, identically or differently,
1. N
2. CR¹;
b) R¹ and R³ are, identically or differently,
1. H
2. halogen
3. (C₁-C₆)-alkyl
4. O-R⁶
5. S-R⁶
6. NHR⁶
7. (C₆-C₁₂)-aryl
8. (C₆-C₁₂)-aryl-(C₁-C₃)-alkyl
9. C(O)-OR⁶
10. C(O)-H
11. (C₂-C₅)-alkenyl
12. NO₂
13. SO₃R⁷
14. CN
15. C(O)-NHR⁸
where 3., 7., 8. and 11. can optionally be substituted by one or more groups such as
C(O)-(O)ₒ-(C₁-C₅)-alkyl, OR⁶, SR⁷, NO₂, CN, NHR⁸ or halogen;
c) R² is a compound of the formula (II) 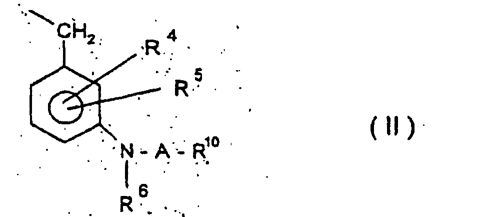
d) R⁴ and R⁵ are, identically or differently,
1. H
2. halogen
3. OR⁶
4. SR⁶
5. CN
6. (C₁-C₅)-alkyl;
e) R⁶, R⁷ and R⁸ are, identically or differently,
1. H
2. (C₁-C₅)-alkyl
3. (C₃-C₅)-alkenyl
4. (C₆-C₁₂)-aryl-(C₁-C₃)-alkyl;
5. (C₃-C₁₀)-cycloalkyl,
6. (C₃-C₁₀)-cycloalkyl-(C₁-C₃)-alkyl;
7. C(O)-(O)ₒ-(C₁-C₅)-alkyl,
8. C(O)-(NH)ₒ-(C₁-C₅)-alkyl;
f) A is an aminocarboxylic acid selected from the group comprising methionine, alanine, phenylalanine, 2-chlorophenylalanine, 3-chlorophenylalanine, 4-chlorophenylalanine, 2-fluorophenylalanine, 3-fluorophenylalanine, 4-fluorophenylalanine, tyrosine, O-methyltyrosine, β-(2-thienyl)alanine, glycine, cyclohexylalanine, leucine, isoleucine, valine, norleucine, phenylglycine, serine, cysteine, aminopropionic acid or aminobutyric acid;
g) R⁹ is
1. H
2. C(O)-(O)ₒ-(C₁-C₅)-alkyl
3. C(O)-(O)ₒ-(C₁-C₃)-alkyl-(C₆-C₁₀)aryl;
h) R¹⁰ is
1. -C(O)-D-E
2. -C(S)-D-E
3. -SO₂-D-E
4. hydrogen;
i) D is
1. (C₂-C₅)-alkenediyl
2. (C₁-C₈)-alkanediyl
3. -(CH₂)ₙ-Yₒ-(CH₂)ₘ-
4. (C₃-C₁₀)-cycloalkanediyl
5. (C₃-C₁₀)-cycloalkyl-(C₁-C₃)-alkanediyl
6. (C₃-C₁₀)-cycloalkenediyl
7. (C₃-C₁₀)-cycloalkenyl-(C₁-C₃)-alkanediyl
where 1.-7. can optionally be substituted by one or more groups such as OR⁶, NO₂, CN, CO₂R⁷, NR⁸R⁹, SO₂R⁶, SO₂NR⁸R⁹, SO₃R⁷ or C(O)-NR⁸R⁹;
j) E is
1. H
2. (C₆-C₁₀)-aryl,
3. (C₁-C₉)-heteroaryl,
where 2. and 3. can optionally be substituted by one or more groups such as NR⁸R⁹, CN, CO₂R⁶, SO₃R⁷, NO₂, SO₂NR⁸R⁹, SO₂R⁶, O-(C₁-C₅)-alkyl, S-(C₁-C₅)-alkyl, (C₁-C₅)-alkyl or (C₂-C₅)-alkenyl, where O-(C₁-C₅)-alkyl and (C₁-C₅)-alkyl can optionally be partially or completely substituted by halogen;
k) Y is
1. O
2. S
3. NR⁸;
l) n and m are, identically or differently, a number 0-6;
m) o is 0 or 1;
and the physiologically tolerated salts thereof.

2. A compound of the formula (I) as claimed in claim 1, in which the symbols have the following meaning:
a) R¹ and R³ are, identically or differently,
1. H
2. (C₁-C₆)-alkyl
3. O-R⁶
4. S-R⁶
5. NHR⁶
6. (C₂-C₅)-alkenyl
7. C(O)-OR⁶
8. C(O)-H
9. NO₂
10. CN
11. C(O)-NHR⁸
where 2. and 6. can optionally be substituted by one or more groups, such as halogen, CO₂R⁶, or NHR⁸ ;
b) R⁶, R⁷ and R⁸ are, identically or differently,
1. H
2. (C₁-C₅)-alkyl
3. (C₆-C₁₀)-aryl-(C₁-C₃)-alkyl.

3. A compound of the formula (I) as claimed in claim 1 or 2, in which
a) R¹ and R³ are, identically or differently,
1. H
2. (C₁-C₄)-alkyl
3. NH-(C₁-C₅)alkyl
4. O-(C₁-C₅)alkyl
5. S-(C₁-C₅)-alkyl
6. C(O)-H
7. CO₂R⁶
8. (C₂-C₃)-alkenyl
where 2.-5. and 8. can be substituted by one or more radicals, such as halogen, CO₂R⁶ or NHR⁸;
b) A is leucine, isoleucine, valine, alanine, methionine, glycine, serine, aminopropionic acid or aminobutyric acid.

4. A process for preparing compounds of the formula (I) as claimed in claims 1 to 3, which comprises
a₁)
1. firstly acylating, at temperatures of between 0 and 20°C, a compound of the formula (III) 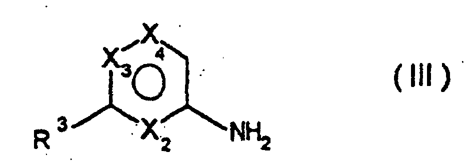 in which R³, X₂, X₃ and X₄ are defined as above in formula I, with activated carboxylic acid derivatives using an auxiliary base,
2. heating the resulting compound of the formula (IV) 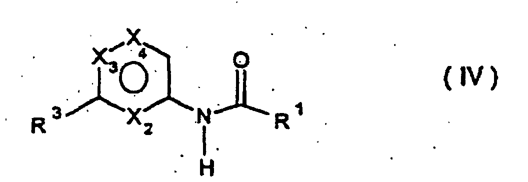 to boiling with Laweson's reagent in butyl acetate or other inert high-boiling point solvents, and thereby obtaining a compound of the formula (V), 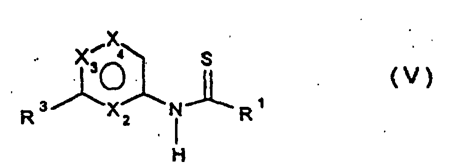 in which R¹, R³, X₂, X₃ and X₄ in formulae IV and V are defined as above in formula I, and where, when X₂ or X₃ is C-O-R², R² is R²' = H or (C₁-C₅)-alkyl,
3. reacting the resulting compound (V), by means of free-radical cyclization, with free-radical-generating reagents, preferably K₃Fe(CN)₆ or Br₂ in inert solvents, at temperatures of between 80 and 110°C, and thereby obtaining a compound of formula (VI) 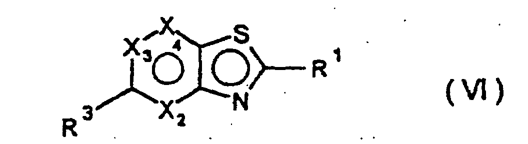 in which the radicals R¹, R³, X₂, X₃ and X₄ are defined as above in formula I, and where, when X₂ or X₃ is C-O-R², R² is R²' = H or (C₁-C₅)-alkyl,
4. converting a compound of the formula (VI), in which X₂ or X₃ is C-O-R² and is defined as under 3., by means of ether-cleaving reagents in inert solvents or without solvent, at temperatures of between 0°C and the boiling point, into compounds of the formula (VI) in which X² or X³ is COR² and R² = R²' = hydrogen;
or
a₂)
1. converting a compound of the formula (VII) 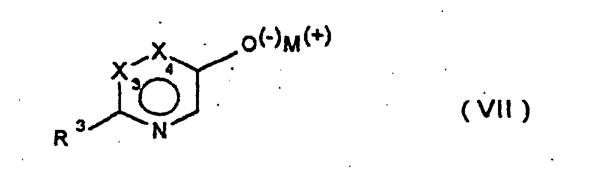 in which R³, X₃, and X₄ are defined as above in formula I and M is potassium, sodium or cesium,
by successive treatment with CO₂ and then NH₃ under elevated pressure and temperatures, preferably 100 atm and 200°C, into a compound of the formula (VIII), 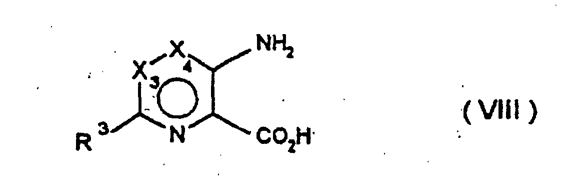 in which R³, X₃ and X₄ are defined as above in formula I,
2. converting a compound of the formula (VIII), by diazotization and subsequent treatment with HS-CHR¹-CO₂H, into a compound of the formula (IX) 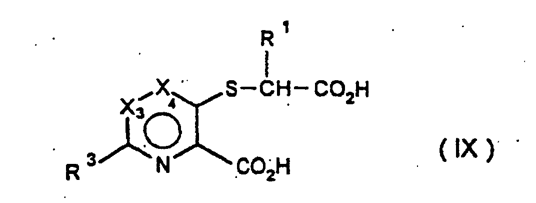 in which R¹, R³, X₃ and X₄ are defined as above in formula I,
3. converting a compound of the formula (IX), by means of cyclization with simultaneous decarboxylation and water elimination, in inert solvents or without solvent into a compound of the formula (X), 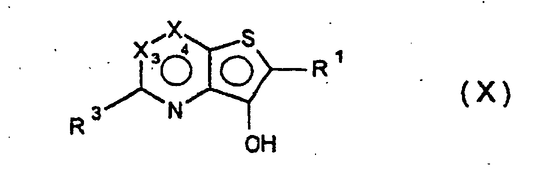 in which R¹, R³, X₃ and X₄ are defined as above in formula (I);
b) deprotonating a compound of the formulae (VI) or (X) 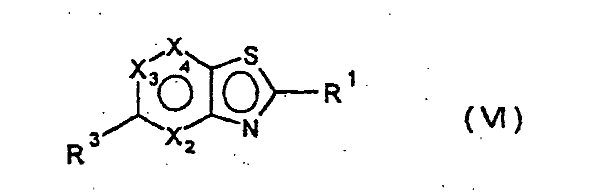 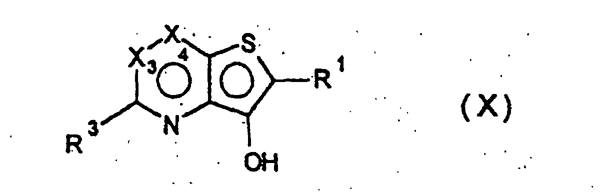 in which X₂, X₃, X₄, R¹ and R³ are defined as above in formula (I), in the case of compounds of the formula (VI), X₂ or X₃ is C-O-H, with Cs₂CO₃ or K₂CO₃ in an inert solvent, and reacting it, at room temperature, with a compound of the formula (Xl) 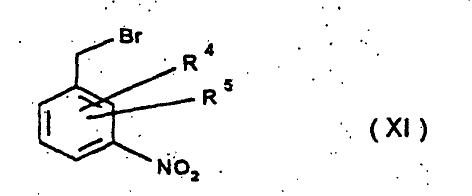 in which R⁴ and R⁵ are defined as above in formula (II);
c) reducing the resulting compounds of the formulae (XII) or (XII') 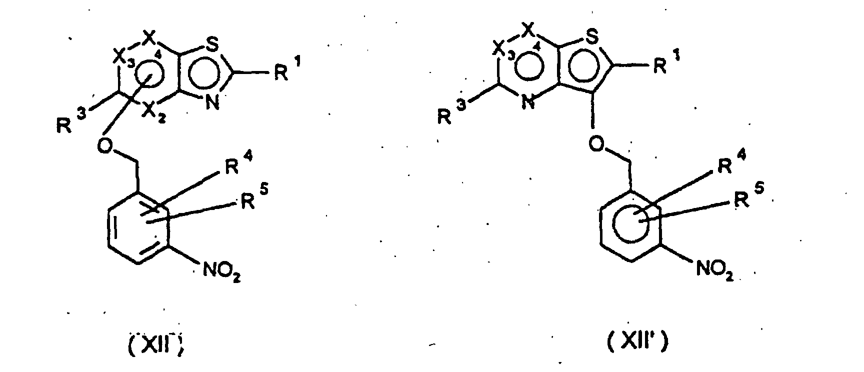 in which R¹, R³, R⁴, R⁵, X₂, X₃ and X₄ are defined as above in formulae (I) and (II), with the aid of transition metal halides to form a compound of the formulae (XIII) or (XIII'), 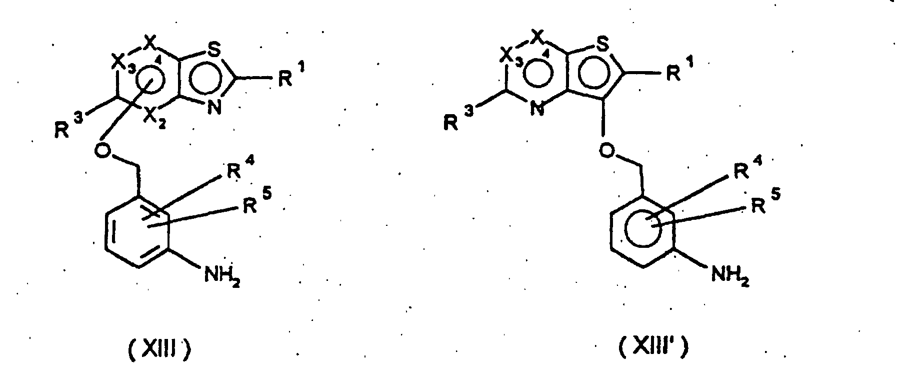 in which R¹, R³, R⁴, R⁵, X₂, X₃ and X₄ are defined as above in formulae (I) and (II);
d) reacting a compound of the formulae (XIII) or (XIII') with activated, suitably protected, aminocarboxylic acid derivatives of A (A-Prot), in inert solvents, where appropriate by adding DMAP, and thereby obtaining a compound of the formulae (XIV) or (XIV'), 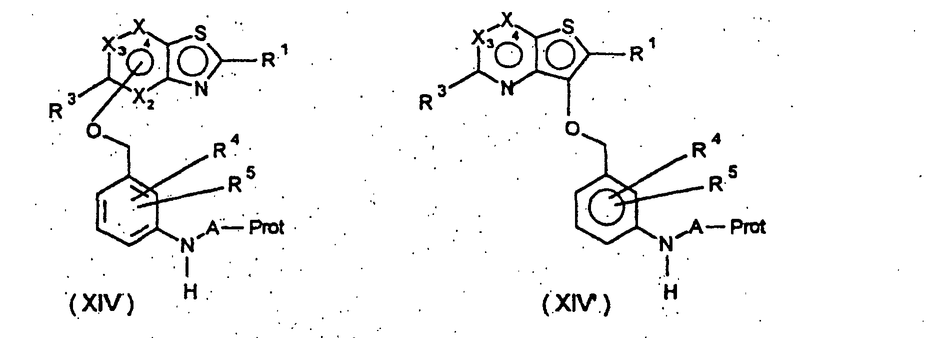 in which A, R¹, R³, R⁴, R⁵, X₂, X₃ and X₄ are defined as above in formulae (I) and (II), and
Prot is an amino protecting group, with both protons of the amino protecting group being protected;
e) reacting a compound of the formulae (XVI) or (XIV'), after alkali metal hydrides, alkali metal carbonates or alcoholates, in inert solvents, have acted on it, with R⁶X, where R⁶ is defined as above in formula (I), with the exception of R⁶=H₅ and X is a leaving group, thereby obtaining a compound of the formulae (XV) or (XV'), 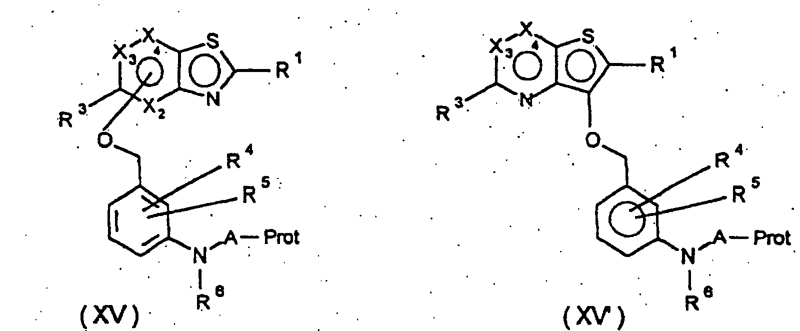 in which A, R¹, R³, R⁴, R⁵, R⁶, X₂, X₃ and X₄ are defined as above in formula (I) and (II), and Prot is defined as above in formula (XIV);
f) for the purpose of removing the protecting group (Prot.) from the compound of the formulae (XV) or (XV'), the latter is preferably reacted with hydrazine, in the case of the phthaloyl group, in alcohols as solvents, at temperatures of between room temperature and the boiling point, thereby affording a compound of the formulae (XVI) or (XVI'), 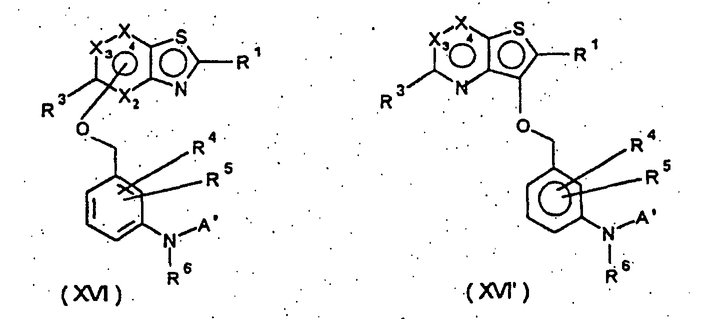 in which R¹, R³, R⁴, R⁵, R⁶, X₂, X₃ and X₄ are defined as above in formulae (I) and (II) and Prot is defined as above in formula (XIV) and A' is a radical of the amino acid A;
g₁) reacting a compound of the formulae (XVI) or (XVI') with activated acid derivatives of the formulae (XVII), (XVIII) or (XIX)
E - D - C(O) - OH (XVII)
E - D - C(S) - OH (XVIII)
E - D - SO₂ - OH (XIX)
in which D and E are defined as above in formula (II),
or
g₂) reacting a compound of the formulae (XVI) or (XVI') with an amine or an alcohol of the formula (XX)
E - D - Z (XX)
in which E and D are defined as above and Z is OH or NH₂, in inert solvents with, however, the compounds of the formulae (XVI), (XVI') or (XX) first being allowed to react with a doubly activated carbonyl compound for the purpose of forming the urea group or urethane group, for example with carbodiimides, phosgene or chlorocarbonates, or
g₃) reacting a compound of the formulae (XVI) or (XVI') with an appropriate isocyanate or isothiocyanate, in inert solvents, and
h) where appropriate, using known methods to convert the resulting compound of the formula (I) into its physiologically tolerated salts.

5. The use of the compounds of the formula (I) as claimed in claims 1 to 3 as medicaments.

6. A medicament comprising at least one compound of the formula (I) as claimed in claims 1 to 4.

## Revendications

1. Composés de formule I 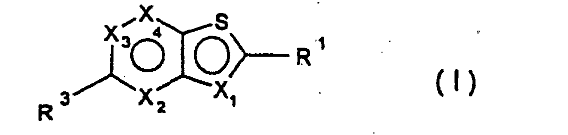 les symboles possèdent les significations suivantes :
a) un des restes X₁, X₂ ou X₃ représente un groupe C-O-R² et chaque fois les autres X₁, X₂, X₃ et X₄ sont identiques ou différents et représentent un groupe
1. N
2. CR¹ ;
b) R¹ et R³ sont identiques ou différents et représentent un groupe
1. hydrogène
2. halogène
3. alkyle en C₁-C₆
4. O-R⁶
5. S-R⁶
6. NHR⁶
7. aryle en C₆-C₁₂
8. (aryl en C₆-C₁₂)-alkyle en C₁-C₃
9. C(O)-OR⁶
10. C(O)-H
11. alcényle en C₂-C₅
12. NO₂
13 SO₃R⁷
14. CN
15. C(O)NHR⁸
3., 7., 8., 11. peuvent éventuellement être substitués par un ou plusieurs groupes tels que C(O)-(O)₀-alkyle en C₁-C₅, OR⁶, SR⁷, NO₂, CN, NHR⁸, halogène ;
c) R² représente un reste de formule (II) 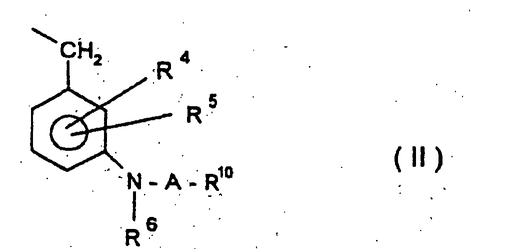
d) R⁴ et R⁵ sont identiques ou différents et représentent un groupe
1. hydrogène
2. halogène
3. O-R⁶
4. S-R⁶
5. CN
6. alkyle en C₁-C₅
e) R⁶' R⁷ et R⁸ sont identiques ou différents et représentent un groupe
1. hydrogène
2. alkyle en C₁-C₅
3. alcényle en C₃-C₅
4. (aryl en C₆-C₁₂)-alkyle en C₁-C₃
5. cycloalkyle en C₃-C₁₀
6. (cycloalkyle en C₃-C₁₀)-(alkyle en C₁-C₃)
7. C(O)-(O)₀-alkyle en C₁-C₅
8. C(O)-(NH)₀-alkyle en C₁-C₅
f) A représente un acide aminocarboxylique pris dans le groupe comprenant la méthionine, l'alanine, la phénylalanine, la 2-chlorophénylalanine, la 3-chlorophénylalanine, la 4-chlorophénylalanine, la 2-fluorophényl-alanine, la 3-fluorophénylalanine, la 4-fluorophénylalanine, la tyrosine, l'O-méthyltyrosine, la β-(2-thiényl)alanine, la glycine, la cyclohexylalanine, la leucine, l'isoleucine, la valine, la norleucine, la phénylglycine, la sérine, la cystéine, l'acide aminopropionique ou l'acide aminobutyrique ;
g) R⁹ représente un groupe
1. hydrogène
2. C(O)-(O)₀-alkyle en C₁-C₅
3. C(O)-(O)₀-alkyl en C₁-C₃)-(aryle en C₆-C₁₀)
h) R¹⁰ représente un groupe
1. -C(O)-D-E
2. -C(S)-D-E
3. -SO₂-D-E
4. hydrogène
i) D représente un groupe
1. (alcène en C₂-C₅)diyle
2. (alcane en C₁-C₈)diyle
3. -(CH₂)ₙ-Yₒ-(CH₂)ₘ-
4. cyclo(alcane en C₃-C₁₀)diyle
5. cyclo(alkyl en C₃-C₁₀)-(alcane en C₁-C₃)diyle
6. cyclo(alcène en C₃-C₁₀)diyle
7. cyclo(alcényl en C₃-C₁₀)-(alcane en C₁-C₃)-diyle
1.-7. pouvant être éventuellement substitués par un ou plusieurs groupes comme par exemple OR⁶, NO₂, CN, CO₂R⁷, NR⁸R⁹, SO₂R⁶, SO₂NR⁸R⁹, SO₃R⁷ ou C(O)-NR⁸R⁹ ;
j) E représente un groupe
1. H
2. aryle en C₆-C₁₀
3. hétéroaryle en C₁-C₉
2. et 3. pouvant éventuellement être substitués par un ou plusieurs groupes comme par exemple NR⁸R⁹, CN, CO₂R⁶, SO₃R⁷, NO₂, SO₂NR⁸R⁹, SO₂R⁶, O-alkyle en C₁-C₅, S-alkyle en C₁-C₅, alkyle en C₁-C₅, alcényle en C₂-C₅, O-alkyle en C₁-C₅ et alkyle en C₁-C₅ pouvant éventuellement être partiellement ou entièrement substitués par des substituants halogènes ;
k) Y représente un groupe
1. O
2. S
3. NR⁸ ;
l) n et m sont identiques ou différents et vont de 0 à 6 ;
m) o vaut 0, 1 ;
ainsi que leurs sels physiologiquement compatibles.

2. Composés de formule (I) selon la revendication 1, dans laquelle les symboles possèdent les significations suivantes :
a) R¹ et R³ sont identiques ou différents et représentent un groupe
1. hydrogène
2. alkyle en C₁-C₆
3. O-R⁶
4. S-R⁶
5. NHR⁶
6. alcényle en C₂-C₅
7. C(O)-OR⁶
8. C(O)-H
9. NO₂
10. CN
11. C(O)NHR⁸
2. et 6. pouvant éventuellement être substitués par un ou plusieurs groupes, comme par exemple halogène, CO₂R⁶, NHR⁸ ;
b) R⁶, R⁷ et R⁸ sont identiques ou différents et représentent un groupe
1. hydrogène
2. alkyle en C₁-C₅
3. (aryl en C₆-C₁₀)-(alkyle en C₁-C₃).

3. Composés de formule (I) selon la revendication 1 ou 2, où
a) R¹ et R³ sont identiques ou différents et représentent un groupe
1. hydrogène
2. alkyle en C₁-C₄
3. NH-(alkyle en C₁-C₅)
4. O-(alkyle en C₁-C₅)
5. S-(alkyle en C₁-C₅)
6. C(O)-H
7. CO₂R⁶
8. alcényle en C₂-C₃ 2.-5. et 8. pouvant éventuellement être substitués par un ou plusieurs restes, comme par exemple halogène, CO₂R⁶, NHR⁸ ;
b) A représente la leucine, l'isoleucine, la valine, l'alanine, la méthionine, la glycine, la sérine, l'acide amino-propionique, l'acide aminobutyrique.

4. Procédé pour la préparation de composés de formule (I) selon les revendications 1 à 3, **caractérisé en ce que**
a₁)
1. un composé de formule (III) 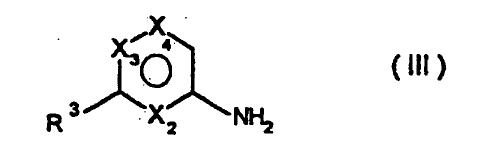 où R³, X₂, X₃ et X₄ sont définis comme ci-dessus,
est d'abord acylé par des dérivés d'acides carboxyliques activés en utilisant une base auxiliaire, à une température comprise entre 0 et 20°C,
2. le composé de formule (IV) ainsi obtenu 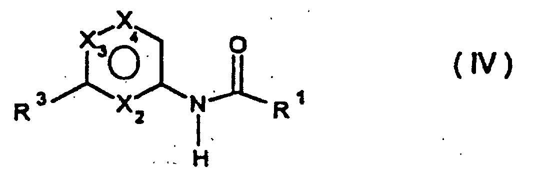 est chauffé à ébullition avec le réactif de Lawesson dans de l'acétate de butyle ou d'autres solvants inertes de haut point d'ébullition et on obtient ainsi un composé de formule (V) 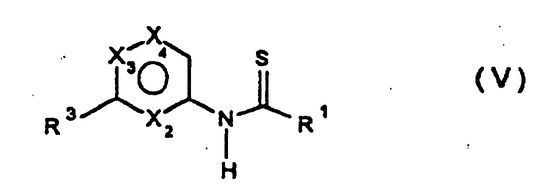 où R¹, R³, X₂, X₃ et X₄ dans les formules IV et V sont définis comme ci-dessus dans la formule I, X₂ ou X₃ = C-O-R², R² représente R^{2'} = H ou alkyle en C₁-C₅,
3. on fait réagir le composé (V) ainsi obtenu par cyclisation radicalaire avec des réactifs engendrant des radicaux, de préférence K₃Fe(CN)₆ ou Br₂ dans des solvants inertes à une température comprise entre 80 et 110°C et ce faisant, on obtient un composé de formule (VI) 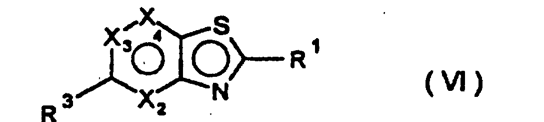 où les restes R¹, R³, X₂, X₃ et X₄ sont définis comme ci-dessus dans la formule I, X₂ ou X₃ = C-O-R², R² représente R²' = H ou alkyle en C₁-C₅,
4. un composé de formule (VI), où X₂ ou X₃ = C-O-R² et sont définis comme au point 3., par des réactifs à coupure éther dans des solvants inertes ou sans solvant à une température comprise entre 0°C et le point d'ébullition, pour obtenir des composés de formule (VI), où X₂ ou X₃ représentent C-O-R² et R² = R^{2'} = hydrogène ;
ou
a2)
1. un composé de formule (VII) 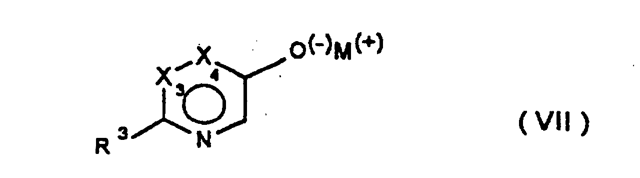 où R³, X₃, X₄ sont définis comme ci-dessus à la formule I et M représente un atome de potassium, de sodium ou de césium, est transformé par traitement successif par CO₂ et ensuite par NH₃ sous pression et température élevées, de préférence 100 atm et 200°C, en un composé de formule (VIII) 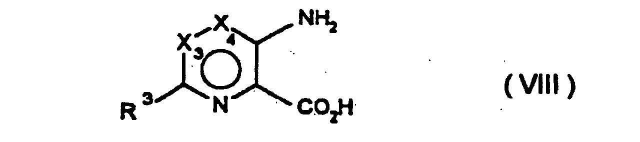 où R³, X₃, X₄ sont définis comme ci-dessus à la formule I,
2. un composé de formule (VIII) est transformé par diazotation et traitement ultérieur par HS-CHR¹-CO₂H en un composé de formule (IX), 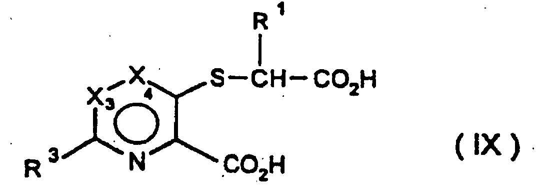 où R¹, R³, X₃, X₄ sont définis comme ci-dessus à la formule I,
3. un composé de formule (IX) est transformé par cyclisation en décarboxylant et en séparant l'eau en même temps dans des solvants inertes ou sans solvants en un composé de formule (X), 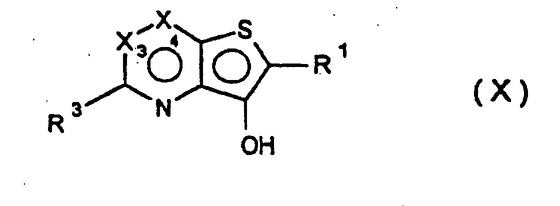 où R¹, R³, X₃, X₄ sont définis comme ci-dessus à la formule I,
b) un composé de formule (VI) ou (X) 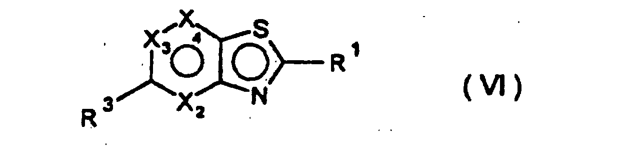 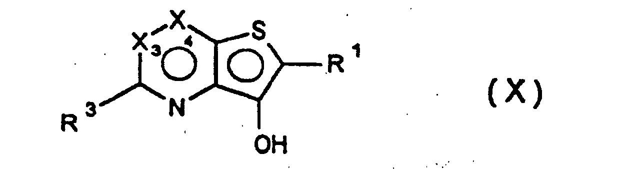 où X₂, X₃, X₄, R¹ et R³ sont définis comme ci-dessus à la formule I, dans le cas de composés de formule (VI) X₂ ou X₃ = C-O-H, est déprotoné par CsCO₃ ou K₂CO₃ dans un solvant inerte et à température ambiante sur un composé de formule (XI) 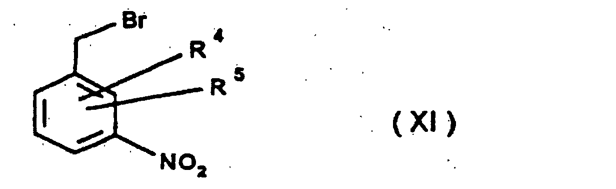 où R⁴ et R⁵ sont définis comme ci-dessus à la formule (II) ;
c) on fait réagir les composés ainsi obtenus de formules (XII) ou (XII') 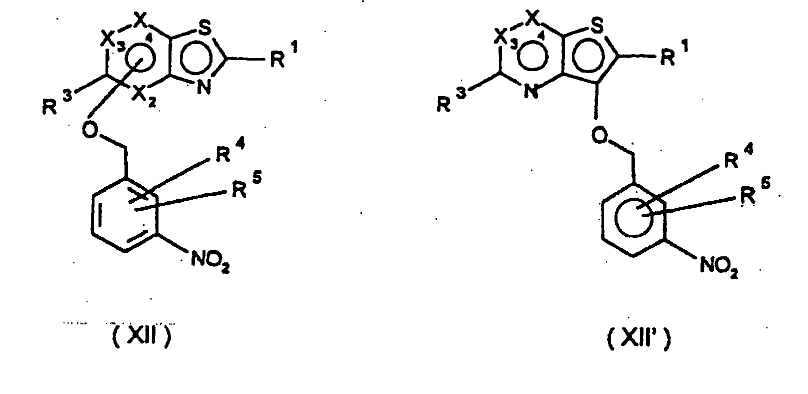 où R¹, R³, R⁴, R⁵, X₂, X₃, X₄ sont définis comme ci-dessus aux formules (I) et (II), on les réduit à l'aide d'halogénures de métaux de transition pour obtenir un composé de formules (XIII) ou (XIII') 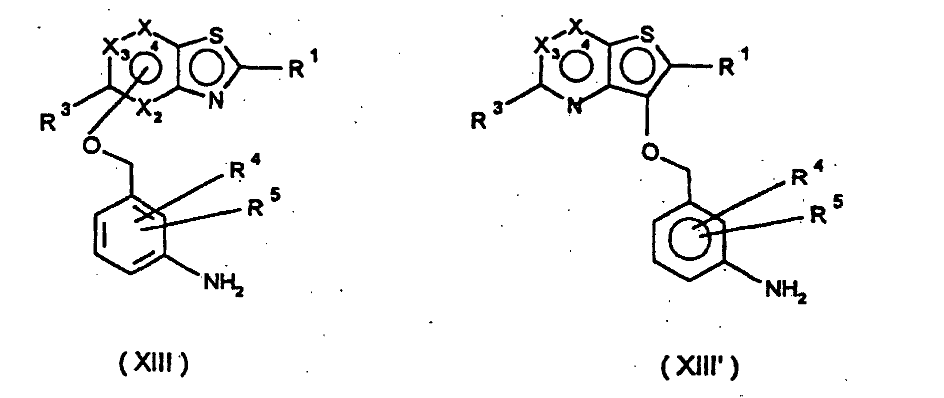 où R¹, R³, R⁴, R⁵, X₂, X₃ et X₄ sont définis comme ci-dessus aux formules (I) et (II) ;
d) on fait réagir un composé de formules (XIII) ou (XIII') sur des dérivés d'acides amino-carboxyliques A (A-Prot) activés, protégés de façon appropriée, dans des solvants inertes, éventuellement par ajout de DMAP, et on obtient ainsi un composé de formules (XIV) ou XIV') 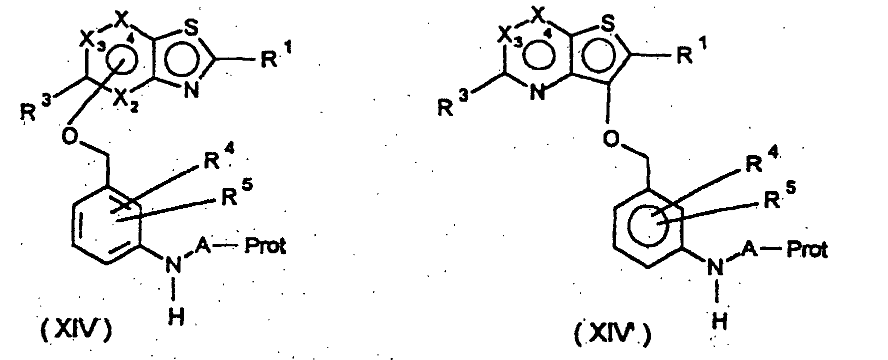 où A, R¹, R³, R⁴, R⁵, X₂, X₃ et X₄ sont définis comme ci-dessus aux formules (I) et (II) et Prot représente un groupe protecteur d'amino, les deux protons du groupe protecteur d'amino étant protégés ;
e) on fait réagir un composé de formules (XIV) ou (XIV') après avoir fait agir des hydrures alcalins, des carbonates alcalins ou des alcoolates dans des solvants inertes, suivie d'un traitement par R⁶X, R⁶ étant défini comme ci-dessus à la formule (I), à l'exception de R⁶ = H, et X représente un groupe partant, en obtenant un composé de formules (XV) ou (XV') 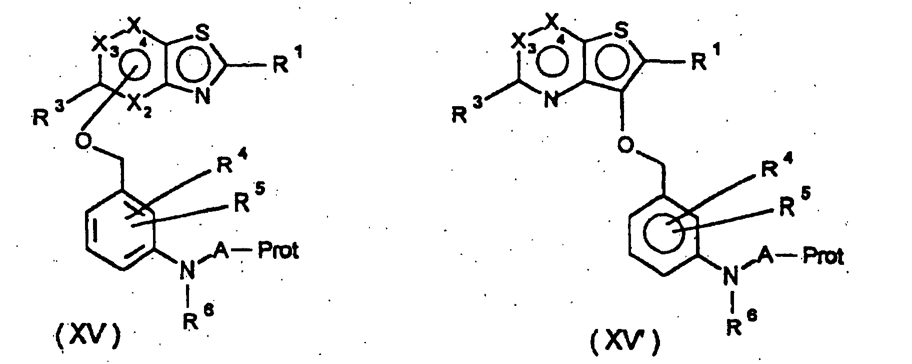 où A, R¹, R³, R⁴, R⁵, X₂, X₃ et X₄ sont définis comme ci-dessus aux formules (I) et (II) et Prot est défini comme ci-dessus à la formule (XIV) ;
f) pour éliminer le groupe protecteur (Prot.) du composé de formules (XV) ou (XV'), dans le cas du groupe phtaloyle de préférence par l'hydrazine dans des alcools comme solvants, à une température, comprise entre la température ambiante et le point d'ébullition, en obtenant un composé de formules (XVI) ou (XVI') 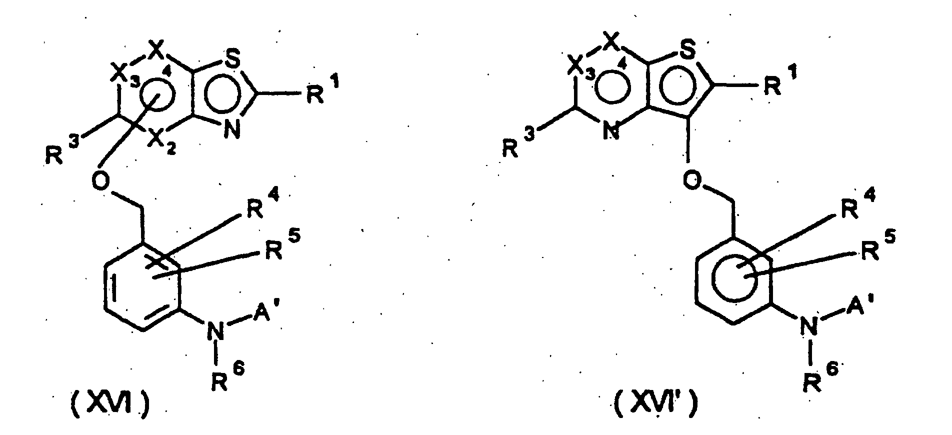 où R¹, R³, R⁴, R⁵, R⁶, X₂, X₃ et X₄ sont définis comme ci-dessus aux formules (I) et (II) et Prot est défini comme ci-dessus à la formule (XIV) et A' représente un reste de l'aminoacide A ;
g₁) on fait réagir un composé de formules (XVI) ou (XVI') sur des dérivés d'acide activés de formules (XVII), (XVIII) ou (XIX)
E-D-C(O)-OH (XVII)
E-D-C(S)-OH (XVIII)
E-D-SO₂-OH (XIX)
où D et E sont définis comme ci-dessus à la formule (II), ou
g₂) on fait réagir un composé de formules (XVI) ou (XVI') sur une amine ou un alcool de formule (XX)
E-D-Z (XX)
où E et D sont définis comme ci-dessus et Z représente un groupe OH ou NH₂, mais en faisant réagir d'abord les composés de formules (XVI), (XVI') ou (XX) sur un composé carbonylé doublement activé en vue de former un groupe urée ou uréthanne, par exemple sur des carbodiimides, le phosgène ou des esters de l'acide chlorocarbonique dans un solvant inerte ou
g3) on fait réagir un composé de formules (XVI) ou (XVI') sur un isocyanate ou isothiocyanate correspondant dans des solvants inertes, et
h) éventuellement on convertit le composé obtenu de formule (I) selon des méthodes connues en leurs sels physiologiquement tolérés.

5. Utilisation des composés de formules (I) selon les revendications 1 à 3 comme médicament.

6. Médicament renfermant au moins un composé de formule (I) selon les revendications 1 à 4.
